# EUROPEAN PATENT APPLICATION

(11) **EP 1 398 029 A1**
(43) Date of publication of application: **17.03.2004**
(21) Application number: 02020256.0
(22) Date of filing: 10.09.2002
(51) Int. Cl.: A61K 31/4155, A61K 31/415, C07D 405/04

(54) **NR3B1 nuclear receptor binding 3-substituted pyrazole derivatives**

(71) Applicant: LION bioscience AG, 69123 Heidelberg (DE)
(72) Inventor: Deuschle, Ulrich, 69245 Bammental (DE); Heck, Stefanie, 69120 Heidelberg (DE); Kober, Ingo, 69251 Gaiberg (DE); Bauer, Ulrike, 69207 Sandhausen (DE); Balogh, Imoia, 69469 Weinheim (DE)
(74) Representative: Goddar, Heinz J., Dr.

(57) **Abstract**

The present invention relates to compounds according to the general formula (1) which bind to the NR3B1 receptor and act as antagonists of the NR3B1 receptor. The invention further relates to the treatment of diseases and/or conditions through binding of said nuclear receptor by said compounds and the production of medicaments using said compounds.

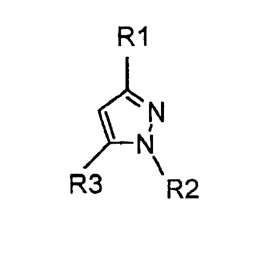

SEQUENCE LISTING

<110>  LION bioscience

<120>  NR3B1 nuclear receptor binding compounds

<130>  FB11719

<160>  9

<170>  PatentIn version 3.1

<210>  1
<211>  1377
<212>  DNA
<213>  Homo sapiens

<400>  1
atggattcgg tagaactttg ccttcctgaa tctttttccc tgcactacga ggaagagctt      60

ctctgcagaa tgtcaaacaa agatcgacac attgattcca gctgttcgtc cttcatcaag     120

acggaacctt ccagcccagc ctccctgacg gacagcgtca accaccacag ccctggtggc     180

tcttcagacg ccagtgggag ctacagttca accatgaatg gccatcagaa cggacttgac     240

tcgccacctc tctacccttc tgctcctatc ctgggaggta gtgggcctgt caggaaactg     300

tatgatgact gctccagcac cattgttgaa gatccccaga ccaagtgtga atacatgctc     360

aactcgatgc ccaagagact gtgtttagtg tgtggtgaca tcgcttctgg gtaccactat     420

ggggtagcat catgtgaagc ctgcaaggca ttcttcaaga ggacaattca aggcaatata     480

gaatacagct gccctgccac gaatgaatgt gaaatcacaa agcgcagacg taaatcctgc     540

caggcttgcc gcttcatgaa gtgtttaaaa gtgggcatgc tgaaagaagg ggtgcgtctt     600

gacagagtac gtggaggtcg gcagaagtac aagcgcagga tagatgcgga gaacagccca     660

tacctgaacc ctcagctggt tcagccagcc aaaaagccat ataacaagat tgtctcacat     720

ttgttggtgg ctgaaccgga gaagatctat gccatgcctg accctactgt ccccgacagt     780

gacatcaaag ccctcactac actgtgtgac ttggccgacc gagagttggt ggttatcatt     840

ggatgggcga agcatattcc aggcttctcc acgctgtccc tggcggacca gatgagcctt     900

ctgcagagtg cttggatgga aattttgatc cttggtgtcg tataccggtc tctttcattt     960

gaggatgaac ttgtctatgc agacgattat ataatggacg aagaccagtc caaattagca    1020

ggccttcttg atctaaataa tgctatcctg cagctggtaa agaaatacaa gagcatgaag    1080

ctggaaaaag aagaatttgt caccctcaaa gctatagctc ttgctaattc agactccatg    1140

cacatagaag atgttgaagc cgttcagaag cttcaggatg tcttacatga agcgctgcag    1200

gattatgaag ctggccagca catggaagac cctcgtcgag ctggcaagat gctgatgaca    1260

ctgccactcc tgaggcagac ctctaccaag gccgtgcagc atttctacaa catcaaacta    1320

gaaggcaaag tcccaatgca caaacttttt ttggaaatgt tggaggccaa ggtctga       1377

<210> 2
<211> 1566
<212> DNA
<213> Homo sapiens

<400> 2
atgggattgg agatgagctc caaggacagc cctggcagtc tggatggaag agcttgggaa 60

gatgctcaga aaccacaaag tgcctggtgc ggtgggagga aaaccagagt gtatgctaca 120

agcagccggc gggcgccgcc gagtgagggg acgcggcgcg gtggggcggc gcggcccgag 180

gaggcggcgg aggaggggcc gcccgcggcc cccggctcac tccggcactc cgggccgctc 240

ggcccccatg cctgcccgac cgcgctgccg gagccccagg tgaccagcgc catgtccagc 300

caggtggtgg gcattgagcc tctctacatc aaggcagagc cggccagccc tgacagtcca 360

aagggttcct cggagacaga gaccgagcct cctgtggccc tggcccctgg tccagctccc 420

actcgctgcc tcccaggcca caaggaagag gaggatgggg agggggctgg gcctggcgag 480

cagggcggtg ggaagctggt gctcagctcc ctgcccaagc gcctctgcct ggtctgtggg 540

gacgtggcct ccggctacca ctatggtgtg gcatcctgtg aggcctgcaa agccttcttc 600

aagaggacca tccaggggag catcgagtac agctgtccgg cctccaacga gtgtgagatc 660

accaagcgga gacgcaaggc ctgccaggcc tgccgcttca ccaagtgcct gcgggtgggc 720

atgctcaagg agggagtgcg cctggaccgc gtccggggtg ggcggcagaa gtacaagcgg 780

cggccggagg tggacccact gcccttcccg ggccccttcc ctgctgggcc cctggcagtc 840

gctggaggcc cccggaagac agcagcccca gtgaatgcac tggtgtctca tctgctggtg 900

gttgagcctg agaagctcta tgccatgcct gaccccgcag gccctgatgg gcacctccca 960

gccgtggcta ccctctgtga cctctttgac cgagagattg tggtcaccat cagctgggcc 1020

aagagcatcc caggcttctc atcgctgtcg ctgtctgacc agatgtcagt actgcagagc 1080

gtgtggatgg aggtgctggt gctgggtgtg gcccagcgct cactgccact gcaggatgag 1140

ctggccttcg ctgaggactt agtcctggat gaagaggggg cacgggcagc tggcctgggg 1200

gaactggggg ctgccctgct gcaactagtg cggcggctgc aggccctgcg gctggagcga 1260

gaggagtatg ttctactaaa ggccttggcc cttgccaatt cagactctgt gcacatcgaa 1320

gatgagccga ggctgtggag cagctgcgag aagctcctgc acgaggccct gctggagtat 1380

gaagccggcc gggctggccc cggagggggt gctgagcggc ggcgggcggg caggctgctg 1440

ctcacgctac cgctcctccg ccagacagcg ggcaaagtgc tggcccattt ctatggggtg 1500

aagctggagg gcaaggtgcc catgcacaag ctgttcttgg agatgctcga ggccatgatg 1560

gactga 1566

<210> 3
<211> 25
<212> PRT
<213> Homo sapiens

<400> 3

Cys Pro Ser Ser His Ser Ser Leu Thr Glu Arg His Lys Ile Leu His
1               5                   10                  15

Arg Leu Leu Gln Glu Gly Ser Pro Ser
            20                  25

<210> 4
<211> 29
<212> DNA
<213> Homo sapiens

<400> 4
tcgaggcgat ttgtcaaggt cacacagta                                    29

<210> 5
<211> 29
<212> DNA
<213> Homo sapiens

<400> 5
tcgatactgt gtgaccttga caaatcgcc                                    29

<210> 6
<211> 500
<212> PRT
<213> Homo sapiens

<400> 6

Met Ser Ser Asp Asp Arg His Leu Gly Ser Ser Cys Gly Ser Phe Ile
1               5                   10                  15

Lys Thr Glu Pro Ser Ser Pro Ser Ser Gly Ile Asp Ala Leu Ser His
            20                  25                  30

His Ser Pro Ser Gly Ser Ser Asp Ala Ser Gly Gly Phe Gly Leu Ala
        35                  40                  45

Leu Gly Thr His Ala Asn Gly Leu Asp Ser Pro Pro Met Phe Ala Gly
    50                  55                  60

Ala Gly Leu Gly Gly Thr Pro Cys Arg Lys Ser Tyr Glu Asp Cys Ala
65                  70                  75                  80

Ser Gly Ile Met Glu Asp Ser Ala Ile Lys Cys Glu Tyr Met Leu Asn
                85                  90                  95

Ala Ile Pro Lys Arg Leu Cys Leu Val Cys Gly Asp Ile Ala Ser Gly
            100                 105                 110

Tyr His Tyr Gly Val Ala Ser Cys Glu Ala Cys Lys Ala Phe Phe Lys
        115                 120                 125

Arg Thr Ile Gln Gly Asn Ile Glu Tyr Ser Cys Pro Ala Thr Asn Glu
    130                 135                 140

Cys Glu Ile Thr Lys Arg Arg Arg Lys Ser Cys Gln Ala Cys Arg Phe
145                 150                 155                 160

Met Lys Cys Leu Lys Val Gly Met Leu Lys Glu Gly Val Arg Leu Asp
                165                 170                 175

Arg Val Arg Gly Gly Arg Gln Lys Tyr Lys Arg Arg Leu Asp Ser Glu
            180                 185                 190

Ser Ser Pro Tyr Leu Ser Leu Gln Ile Ser Pro Pro Ala Lys Lys Pro
        195                 200                 205

Leu Thr Lys Ile Val Ser Tyr Leu Leu Val Ala Glu Pro Asp Lys Leu
    210                 215                 220

Tyr Ala Met Pro Pro Pro Gly Met Pro Glu Gly Asp Ile Lys Ala Leu
225                 230                 235                 240

Thr Thr Leu Cys Asp Leu Ala Asp Arg Glu Leu Val Val Ile Ile Gly
                245                 250                 255

Trp Ala Lys His Ile Pro Gly Phe Ser Ser Leu Ser Leu Gly Asp Gln
            260                 265                 270

Met Ser Leu Leu Gln Ser Ala Trp Met Glu Ile Leu Ile Leu Gly Ile
        275                 280                 285

Val Tyr Arg Ser Leu Pro Tyr Asp Asp Lys Leu Val Tyr Ala Glu Asp
    290                 295                 300

Tyr Ile Met Asp Glu Glu His Ser Arg Leu Ala Gly Leu Leu Glu Leu
305                 310                 315                 320

Tyr Arg Ala Ile Leu Gln Leu Val Arg Arg Tyr Lys Lys Leu Lys Val
                325                 330                 335

Glu Lys Glu Glu Phe Val Thr Leu Lys Ala Leu Ala Leu Ala Asn Ser
            340                 345                 350

Asp Ser Met Tyr Ile Glu Asp Leu Glu Ala Val Gln Lys Leu Gln Asp
        355                 360                 365

Leu Leu His Glu Ala Leu Gln Asp Tyr Glu Leu Ser Gln Arg His Glu
    370                 375                 380

Glu Pro Trp Arg Thr Gly Lys Leu Leu Leu Thr Leu Pro Leu Leu Arg
385                 390                 395                 400

Gln Thr Ala Ala Lys Ala Val Gln His Phe Tyr Ser Val Lys Leu Gln
                405                 410                 415

Gly Lys Val Pro Met His Lys Leu Phe Leu Glu Met Leu Glu Ala Lys
            420                 425                 430

Ala Trp Ala Arg Ala Asp Ser Leu Gln Glu Trp Arg Pro Leu Glu Gln
        435                 440                 445

Val Pro Ser Pro Leu His Arg Ala Thr Lys Arg Gln His Val His Phe
    450                 455                 460

Leu Thr Pro Leu Pro Pro Pro Pro Ser Val Ala Trp Val Gly Thr Ala
465                 470                 475                 480

Gln Ala Gly Tyr His Leu Glu Val Phe Leu Pro Gln Arg Ala Gly Trp
                485                 490                 495

Pro Arg Ala Ala
            500

<210>  7
<211>  1503
<212>  DNA
<213>  Homo sapiens

<400>  7
atgtcctcgg acgacaggca cctgggctcc agctgcggct ccttcatcaa gactgagccg      60

tccagcccgt cctcgggcat agatgccctc agccaccaca gccccagtgg ctcgtccgac     120

gccagcggcg gctttggcct ggccctgggc acccacgcca acggtctgga ctcgccaccc     180

atgtttgcag gcgccgggct gggaggcacc ccatgccgca agagctacga ggactgtgcc     240

agcggcatca tggaggactc ggccatcaag tgcgagtaca tgctcaacgc catccccaag     300

cgcctgtgcc tcgtg[?]gcgg ggacattgcc tctggctacc actacggcgt ggcctcctgc     360

gaggcttgca aggccttctt caagaggact atccaaggga acattgagta cagctgcccg     420

gccaccaacg agtgcgagat caccaaacgg aggcgcaagt cctgccaggc ctgccgcttc     480

atgaaatgcc tcaaagtggg gatgctgaag gaaggtgtgc gccttgatcg agtgcgtgga 540

ggccgtcaga aatacaagcg acggctggac tcagagagca gcccatacct gagcttacaa 600

atttctccac ctgctaaaaa gccattgacc aagattgtct catacctact ggtggctgag 660

ccggacaagc tctatg[?]cat gcctcccct ggtatgcctg aggggacat caaggccctg 720

accactctct gtgacctggc agaccgagag cttgtggtca tcattggctg ggccaagcac 780

atcccaggct tctcaagcct ctccctgggg gaccagatga gcctgctgca gagtgcctgg 840

atggaaatcc tcatcctggg catcgtgtac cgctcgctgc cctacgacga caagctggtg 900

tacgctgagg actacatcat ggatgaggag cactcccgcc tcgcggggct gctggagctc 960

taccgggcca tcctgcagct ggtacgcagg tacaagaagc tcaaggtgga gaaggaggag 1020

tttgtgacgc tcaaggccct ggccctcgcc aactccgatt ccatgtacat cgaggatcta 1080

gaggctgtcc agaagctgca ggacctgctg cacgaggcac tgcaggacta cgagctgagc 1140

cagcgccatg aggagccctg gaggacgggc aagctgctgc tgacactgcc gctgctgcgg 1200

cagacggccg ccaaggccgt gcagcacttc tatagcgtca aactgcaggg caaagtgccc 1260

atgcacaaac tcttcctgga gatgctggag gccaaggcct gggccagggc tgactccctt 1320

caggagtgga ggccactgga gcaagtgccc tctcccctcc accgagccac caagaggcag 1380

catgtgcatt tcctaactcc cttgccccct cccccatctg tggcctgggt gggcactgct 1440

caggctggat accacctgga ggttttcctt ccgcagaggg caggttggcc aagagcagct 1500

tag 1503

<210> 8
<211> 436
<212> PRT
<213> Homo sapiens

<400> 8
   Met Ser Asn Lys Asp Arg His Ile Asp Ser Ser Cys Ser Ser Phe Ile
   1 5 10 15
   Lys Thr Glu Pro Ser Ser Pro Ala Ser Leu Thr Asp Ser Val Asn His
   20 25 30
   His Ser Pro Gly Gly Ser Ser Asp Ala Ser Gly Ser Tyr Ser Ser Thr
   35 40 45
   Met Asn Gly His Gln Asn Gly Leu Asp Ser Pro Pro Leu Tyr Pro Ser
   50 55 60
   Ala Pro Ile Leu Gly Gly Ser Gly Pro Val Arg Lys Leu Tyr Asp Asp
   65 70 75 80
   Cys Ser Ser Thr Ile Val Glu Asp Pro Gln Thr Lys Cys Glu Tyr Met
                   85                  90                  95
   Leu Asn Ser Met Pro Lys Arg Leu Cys Leu Val Cys Gly Asp Ile Ala
               100                 105                 110
   Ser Gly Tyr His Tyr Gly Val Ala Ser Cys Glu Ala Cys Lys Ala Ser
           115                 120                 125
   Phe Lys Arg Lys Ile Gln Ala Asn Ile Glu Tyr Ser Cys Pro Ala Thr
       130                 135                 140
   Asn Glu Cys Glu Ile Thr Lys Arg Arg Arg Lys Ser Cys Gln Ala Cys
   145                 150                 155                 160
   Arg Phe Met Lys Cys Leu Lys Val Gly Met Leu Lys Glu Gly Val Arg
                   165                 170                 175
   Leu Asp Arg Val Arg Gly Gly Arg Gln Lys Tyr Lys Arg Arg Ile Asp
               180                 185                 190
   Ala Glu Asn Ser Pro Tyr Leu Asn Pro Gln Leu Val Gln Pro Ala Lys
           195                 200                 205
   Lys Pro Tyr Asn Lys Ile Val Ser His Leu Leu Val Ala Glu Pro Glu
       210                 215                 220
   Lys Ile Tyr Ala Met Pro Asp Pro Thr Val Pro Asp Ser Asp Ile Lys
   225                 230                 235                 240
   Ala Leu Thr Thr Leu Cys Asp Cys Ala Asp Arg Glu Leu Val Val Ile
                   245                 250                 255
   Ile Gly Trp Ala Lys His Ile Pro Gly Phe Ser Thr Leu Ser Leu Ala
               260                 265                 270
   Asp Gln Met Ser Leu Leu Gln Ser Ala Trp Met Glu Ile Leu Ile Leu
           275                 280                 285
   Gly Phe Val Tyr Arg Ser Leu Ser Phe Glu Asp Glu Leu Val Tyr Ala
       290                 295                 300
   Asp Asp Tyr Ile Met Asp Glu Asp Gln Ser Lys Leu Ala Gly Leu Leu
   305                 310                 315                 320
   Asp Leu Asn Asn Ala Ile Leu Gln Leu Val Lys Lys Tyr Lys Ser Met
   325 330 335
   Lys Leu Glu Lys Glu Glu Phe Val Thr Leu Lys Ala Ile Ala Leu Ala
   340 345 350
   Asn Ser Asp Ser Met His Ile Glu Asp Val Glu Ala Val Gln Lys Leu
   355 360 365
   Gln Asp Val Leu His Glu Ala Leu Gln Asp Tyr Glu Ala Gly Gln His
   370 375 380
   Met Glu Asp Pro Arg Arg Ala Gly Lys Met Leu Met Thr Leu Pro Leu
   385 390 395 400
   Leu Arg Gln Thr Ser Thr Lys Ala Val Gln His Phe Tyr Asn Ile Lys
   405 410 415
   Leu Glu Gly Lys Val Pro Met His Lys Leu Phe Leu Glu Met Leu Glu
   420 425 430
   Ala Lys Val Cys
   435

<210> 9
<211> 519
<212> PRT
<213> Homo sapiens

<400> 9

Met Gly Leu Glu Met Ser Ser Lys Asp Ser Pro Gly Ser Leu Asp Gly
1               5                   10                  15

Arg Ala Trp Glu Asp Ala Gln Lys Pro Gln Ser Ala Trp Cys Gly Gly
            20                  25                  30

Arg Lys Thr Arg Val Tyr Ala Thr Ser Ser Arg Arg Ala Pro Pro Ser
        35                  40                  45

Glu Gly Thr Arg Arg Gly Gly Ala Ala Arg Pro Glu Glu Ala Ala Glu
    50                  55                  60

Glu Gly Pro Pro Ala Ala Pro Gly Ser Leu Arg His Ser Gly Pro Leu
65                  70                  75                  80

Gly Pro His Ala Cys Pro Thr Ala Leu Pro Glu Pro Gln Val Thr Ser
                85                  90                  95

Ala Met Ser Ser Gln Val Val Gly Ile Glu Pro Leu Tyr Ile Lys Ala

            100                 105                 110

Glu Pro Ala Ser Pro Asp Ser Pro Lys Gly Ser Ser Glu Thr Glu Thr
        115                 120                 125

Glu Pro Pro Val Ala Leu Ala Pro Gly Pro Ala Pro Thr Arg Cys Leu
    130                 135                 140

Pro Gly His Lys Glu Glu Glu Asp Gly Glu Gly Ala Gly Pro Gly Glu
145                 150                 155                 160

Gln Gly Gly Gly Lys Leu Val Leu Ser Ser Leu Pro Lys Arg Leu Cys
                165                 170                 175

Leu Val Cys Gly Asp Val Ala Ser Gly Tyr His Tyr Gly Val Ala Ser
            180                 185                 190

Cys Glu Ala Cys Lys Ala Phe Phe Lys Arg Thr Ile Gln Gly Ser Ile
        195                 200                 205

Glu Tyr Ser Cys Pro Ala Ser Asn Glu Cys Glu Ile Thr Lys Arg Arg
    210                 215                 220

Arg Lys Ala Cys Gln Ala Cys Arg Phe Thr Lys Cys Leu Arg Val Gly
225                 230                 235                 240

Met Leu Lys Glu Gly Val Arg Leu Asp Arg Val Arg Gly Gly Arg Gln
                245                 250                 255

Lys Tyr Lys Arg Arg Pro Glu Val Asp Pro Leu Pro Phe Pro Gly Pro
            260                 265                 270

Phe Pro Ala Gly Pro Leu Ala Val Ala Gly Gly Pro Arg Lys Thr Ala
        275                 280                 285

Pro Val Asn Ala Leu Val Ser His Leu Leu Val Val Glu Pro Glu Lys
    290                 295                 300

Leu Tyr Ala Met Pro Asp Pro Ala Gly Pro Asp Gly His Leu Pro Ala
305                 310                 315                 320

Val Ala Thr Leu Cys Asp Leu Phe Asp Arg Glu Ile Val Val Thr Ile
                325                 330                 335

Ser Trp Ala Lys Ser Ile Pro Gly Phe Ser Ser Leu Ser Leu Ser Asp
            340                 345                 350

Gln Met Ser Val Leu Gln Ser Val Trp Met Glu Val Leu Val Leu Gly
        355                 360                 365

Val Ala Gln Arg Ser Leu Pro Leu Gln Asp Glu Leu Ala Phe Ala Glu
    370                 375                 380

Asp Leu Val Leu Asp Glu Glu Gly Ala Arg Ala Ala Gly Leu Gly Glu
385                 390                 395                 400

Leu Gly Ala Ala Leu Leu Gln Leu Val Arg Arg Leu Gln Ala Leu Arg
                405                 410                 415

Leu Glu Arg Glu Glu Tyr Val Leu Leu Lys Ala Leu Ala Leu Ala Asn
            420                 425                 430

Ser Asp Ser Val His Ile Glu Asp Ala Glu Ala Val Glu Gln Leu Arg
        435                 440                 445

Glu Ala Leu His Glu Ala Leu Leu Glu Tyr Glu Ala Gly Arg Ala Gly
    450                 455                 460

Pro Gly Gly Gly Ala Glu Arg Arg Arg Ala Gly Arg Leu Leu Leu Thr
465                 470                 475                 480

Leu Pro Leu Leu Arg Gln Thr Ala Gly Lys Val Leu Ala His Phe Tyr
                485                 490                 495

Gly Val Lys Leu Glu Gly Lys Val Pro Met His Lys Leu Phe Leu Glu
            500                 505                 510

Met Leu Glu Ala Met Met Asp
        515

## Description

The present invention relates to compounds according to the general formula (1) which bind to the NR3B1 receptor and act as antagonists or agonists of the NR3B1 receptor. The invention further relates to the treatment of diseases and/or conditions through binding of said nuclear receptor by said compounds and the production of medicaments using said compounds.

### BACKGROUND OF THE INVENTION

Multicellular organisms are dependent on advanced mechanisms of information transfer between cells and body compartments. The information that is transmitted can be highly complex and can result in the alteration of genetic programs involved in cellular differentiation, proliferation, or reproduction. The signals, or hormones, are often simple molecules, such as peptides, fatty acid, or cholesterol derivatives.

Many of these signals produce their effects by ultimately changing the transcription of specific genes. One well-studied group of proteins that mediate a cell's response to a variety of signals is the family of transcription factors known as nuclear receptors, hereinafter referred to often as "NR". Members of this group include receptors for steroid hormones, vitamin D, ecdysone, cis and trans retinoic acid, thyroid hormone, bile acids, cholesterol-derivatives, fatty acids (and other peroxisomal proliferators), as well as so-called orphan receptors, proteins that are structurally similar to other members of this group, but for which no ligands are known (Escriva, H. et al., Ligand binding was acquired during evolution of nuclear receptors, PNAS, 94, 6803 - 6808, 1997). Orphan receptors may be indicative of unknown signalling pathways in the cell or may be nuclear receptors that function without ligand activation. The activation of transcription by some of these orphan receptors may occur in the absence of an exogenous ligand and/or through signal transduction pathways originating from the cell surface (Mangelsdorf, D. J. et al., The nuclear receptor superfamily: the second decade, Cell 83, 835-839, 1995).

In general, three functional domains have been defined in NRs. An amino terminal domain is believed to have some regulatory function. A DNA-binding domain hereinafter referred to as "DBD" usually comprises two zinc finger elements and recognises a specific Hormone Responsive Element hereinafter referred to as "HRE" within the promoters of responsive genes. Specific amino acid residues in the "DBD" have been shown to confer DNA sequence binding specificity (Schena, M. & Yamamoto, K.R., Mammalian Glucocorticoid Receptor Derivatives Enhance Transcription in Yeast, Science, 241:965-967, 1988). A Ligand-binding-domain hereinafter referred to as "LBD" is at the carboxy-terminal region of known NRs. In the absence of hormone, the LBD appears to interfere with the interaction of the DBD with its HRE. Hormone binding seems to result in a conformational change in the NR and thus opens this interference (Brzozowski et al., Molecular basis of agonism and antagonism in the oestogen receptor, Nature, 389, 753 - 758, 1997; Wagner et al., A structural role for hormone in the thyroid hormone receptor, Nature, 378, 690 - 697. 1995). A NR without the HBD constitutively activates transcription but at a low level.

Coactivators or transcriptional activators are proposed to bridge between sequence specific transcription factors, the basal transcription machinery and in addition to influence the chromatin structure of a target cell. Several proteins like SRC-1, ACTR, and Grip1 interact with NRs in a ligand enhanced manner (Heery et al., A signature motif in transcriptional coactivators mediates binding to nuclear receptors, Nature, 387, 733 - 736; Heinzel et al., A complex containing N-CoR, mSin3 and histone deacetylase mediates transcriptional repression, Nature 387, 43 - 47, 1997). Furthermore, the physical interaction with negative receptor-interacting proteins or corepressors has been demonstrated (Xu et al., Coactivator and Corepressor complexes in nuclear receptor function, Curr Opin Genet Dev, 9 (2), 140 - 147, 1999).

Nuclear receptor modulators like steroid hormones affect the growth and function of specific cells by binding to intracellular receptors and forming nuclear receptor-ligand complexes. Nuclear receptor-hormone complexes then interact with a hormone response element (HRE) in the control region of specific genes and alter specific gene expression.

The Estrogen Related Receptor (ERR) alpha, beta and gamma (hereinafter referred to as NR3B 1, NR3B2 and NR3B3 when referring to the human receptor) are nuclear receptors which activate genes upon binding to the promoter region of target genes either in a homodimeric or monomeric fashion or as a heterodimer with the estrogen receptor alpha. NR3B1 and NR3B2 were the first orphan nuclear receptors identified more than a decade ago (Giguere, et al. 1988 Nature 331, 91-94). Although the ERR's display high homology to the estrogen receptor (ER) they do not bind or respond to natural occurring estrogens. The ERR's are structurally and functionally related to the ERa and ERb and have been shown to posses the potential to positively and negatively regulate estrogen regulated gene networks (Vanacker, JM et al 1999 EMBO J. 18, 4270-4279).

The ERR's are described as constitutive activators of transcription. They contain a well conserved AF-2 domain that is necessary for the constitutive transcriptional activity. The interaction of the ERR's with cofactors (e.g. GRIP-1, SRC-1, ACTR) is ligand independent. Nevertheless, the observation that the constitutive activity depends on a factor present in serum that can be withdrawn gives a hint, that there may exist also agonistic ligands for the ERR's that can induce their activity.

To date no physiological ligands have been identified for ERRa (NR3B1) although in very recent publications, Diethylstilbestrol was identified as a synthetic substance able to bind to and inhibit the constitutive activity of ERRa but also ERRb and ERRg (Tremblay, G.B. 2001 Genes & Development 15, 833-838).

To date only very few compounds have been described which bind the NR3B1 receptor and thus show utility for treating diseases or conditions which are due to or are influenced by said nuclear receptor (Maloney at al., J Med Chem, 10; 43(16):2971-4, 2000).

It was thus an object of the present invention to provide for novel NR3B1 binding compounds. It was thus an object of the present invention to provide for compounds which by means of binding the NR3B1 receptor act as antagonist or agonist of said receptor and thus show utility for treating diseases or conditions which are due to or influenced by said nuclear receptor.

It was further an object of the invention to provide for compounds which may be used for the manufacture of a medicament for the treatment of conditions or diseases like cancer, osteoporosis, obesity, lipid disorders, cardiovascular disorders or fertility and reproductive health associated conditions or diseases. In a preferred embodiment of the invention it was an object of the invention to provide for compounds for the manufacture of anti-tumour medicaments.

### SUMMARY OF THE INVENTION

The present invention provides, *inter* alia, novel NR3B1 nuclear receptor protein binding compounds according to the general formula (1) shown below. Said compounds are also binders of mammalian homologues of said receptor. Further the object of the invention was solved by providing for amongst the NR3B1 nuclear receptor protein binding compounds according to the general formula (1) such compounds which act as antagonists and such compounds which act as agonists of the human ERRa receptor or a mammalian homologue thereof.

The invention provides for ERRa antagonists or agonists which may be used for the manufacture of a medicament for the treatment of cancer, osteoporosis, obesity, lipid disorders or a cardiovascular disorder or fertility and reproductive health associated conditions or diseases. In a preferred embodiment of the invention the compounds according to the invention may be used for manufacture of anti-tumour medicaments and/or for the treatment of diseases such as cancer.

The foregoing merely summarises certain aspects of the present invention and is not intended, nor should it be construed, to limit the invention in any manner. All patents and other publications recited herein are hereby incorporated by reference in their entirety.

### DETAILED DESCRIPTION OF THE PREFERRED EMBODIMENTS

The invention provides for a compound according to formula (1), or pharmaceutical acceptable salts or solvates thereof, hereinafter also referred to as the "compounds according to the invention" including particular and preferred embodiments thereof, 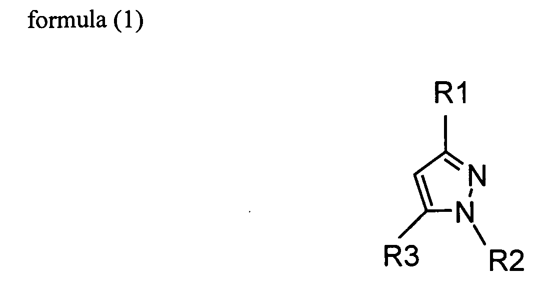 wherein in formula (1) as shown above,
R₁ is phenyl, substituted phenyl, C₅ to C₆ heteroaryl, C₅ to C₆ substituted heteroaryl, napthyl or substituted napthyl,
R₂ is H, C₁ to C₈ alkyl, C₁ to C₇ acyl or C₁ to C₇ substituted acyl, C₁ to C₈ substituted alkyl, C₇ to C₁₂ alkylphenyl or C₇ to C₁₂ substituted phenylalkyl, C₃ to Cg cycloalkyl, C₃ to C₈ substituted cycloalkyl,, C₅ to C₆ heteroaryl, [C₅ to C₆]-heteroaryl-(C₁ to C₆)-alkyl, and
R₃ is H, C₁ to C₈ alkyl, C₁ to C₈ substituted alkyl, C₇ to C₁₂ alkylphenyl or or C₇ to C₁₂ substituted phenylalkyl, halogen, C₁ to C₈ alkoxy, furanyl, substituted furanyl, thiazyl, substituted thiazyl, carboxy, ester, amide or C₁ to C₈ aminoacyl.

The inventors have unexpectedly identified the compounds as well as the general structure capable of effectively binding ERRa and as claimed in the present invention amongst approximately 4500 compounds that were within a compound library that was not previously disclosed

The compounds of the invention can also exist as solvates and hydrates. Thus, these compounds may crystallise with, for example, waters of hydration, or one, a number of, or any fraction thereof of molecules of the mother liquor solvent. The solvates and hydrates of such compounds are included within the scope of this invention.

The term "halogen" refers to the fluoro, chloro, bromo or iodo atoms. There can be one or more halogen, which are the same or different. Preferred halogens are chloro and fluoro.

The symbol "H" denotes a hydrogen atom.

The term "C₁ to C₇ acyl" encompasses groups such as formyl, acetyl, propionyl, butyryl, pentanoyl, pivaloyl, hexanoyl, heptanoyl, benzoyl and the like. Preferred acyl groups are acetyl and benzoyl.

The term "C₁ to C₇ substituted acyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, C₁ to C₆ alkyl ester, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted acyl groups may be substituted once or more, and preferably once or twice, with the same or with different substituents.

Examples of C₁ to C₇ substituted acyl groups include 4-phenylbutyroyl, 3-phenylbutyroyl, 3-phenylpropanoyl, 2- cyclohexanylacetyl, cyclohexanecarbonyl, 2-furamoyl and 3-dimethylaminobenzoyl and the like.

The term "substituted phenyl" specifies a phenyl group substituted with one or more, and preferably one or two, moieties chosen from the groups consisting of halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, wherein the phenyl is substituted or unsubstituted, such that, for example, a biphenyl results.

Examples of the term "substituted phenyl" includes a mono- or di(halo)phenyl group such as 2, 3 or 4-chlorophenyl, 2,6-dichlorophenyl, 2,5-dichlorophenyl, 3,4-dichlorophenyl, 2, 3 or 4-bromophenyl, 3,4-dibromophenyl, 3-chloro-4-fluorophenyl, 2, 3 or 4-fluorophenyl and the like; a mono or di(hydroxy)phenyl group such as 2, 3 or 4-hydroxyphenyl, 2,4-dihydroxyphenyl, the protected-hydroxy derivatives thereof and the like; a nitrophenyl group such as 2, 3 or 4-nitrophenyl; a cyanophenyl group, for example, 2, 3 or 4-cyanophenyl; a mono- or di(alkyl)phenyl group such as 2, 3 or 4-methylphenyl, 2,4-dimethylphenyl, 2, 3 or 4-(iso-propyl)phenyl, 2, 3 or 4-ethylphenyl, 2, 3 or 4-(n-propyl)phenyl and the like; a mono or di(alkoxyl)phenyl group, for example, 2,6-dimethoxyphenyl, 2, 3 or 4-methoxyphenyl, 2, 3 or 4-ethoxyphenyl, 2, 3 or 4-(isopropoxy)phenyl, 2, 3 or 4-(t-butoxy)phenyl, 3-ethoxy-4-methoxyphenyl and the like; 2, 3 or 4-trifluoromethylphenyl; a mono- or dicarboxyphenyl or (protected carboxy)phenyl group such as 2, 3 or 4-carboxyphenyl or 2,4-di(protected carboxy)phenyl; a mono-or di(hydroxymethyl)phenyl or (protected hydroxymethyl)phenyl such as 2, 3, or 4-(protected hydroxymethyl)phenyl or 3,4-di(hydroxymethyl)phenyl; a mono- or di(aminomethyl)phenyl or (protected aminomethyl)phenyl such as 2, 3 or 4-(aminomethyl)phenyl or 2,4-(protected aminomethyl)phenyl; or a mono- or di(N-(methylsulfonylamino))phenyl such as 2, 3 or 4-(N-(methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy 4-chlorophenyl and the like.

The term "heteroaryl" means a heterocyclic aromatic derivative which is a five-membered or six-membered ring system having from 1 to 4 heteroatoms, such as oxygen, sulfur and/or nitrogen, in particular nitrogen, either alone or in conjunction with sulfur or oxygen ring atoms. Examples of heteroaryls include pyridinyl, pyrimidinyl, and pyrazinyl, pyridazinyl, pyrrolo, furano, thiopheno, oxazolo, isoxazolo, phthalimido, thiazolo and the like.

The term "substituted heteroaryl" means the above-described heteroaryl is substituted with, for example, one or more, and preferably one or two, substituents which are the same or different which substituents can be halogen, hydroxy, protected hydroxy, cyano, nitro, C₁ to C₆ alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino groups.

The term "substituted naphthyl" specifies a naphthyl group substituted with one or more, and preferably one or two, moieties either on the same ring or on different rings chosen from the groups consisting of halogen, hydroxy, protected hydroxy, cyano, nitro, C₁ to C₆ alkyl, C₁ to C₇ alkoxy, C₁ to C₇ acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino.

Examples of the term "substituted naphthyl" includes a mono or di(halo)naphthyl group such as 1, 2, 3, 4, 5, 6, 7 or 8-chloronaphthyl, 2, 6-dichloronaphthyl, 2, 5-dichloronaphthyl, 3, 4-dichloronaphthyl, 1, 2, 3, 4, 5, 6, 7 or 8-bromonaphthyl, 3, 4-dibromonaphthyl, 3-chloro-4-fluoronaphthyl, 1, 2, 3, 4, 5, 6, 7 or 8-fluoronaphthyl and the like; a mono or di(hydroxy)naphthyl group such as 1, 2, 3, 4, 5, 6, 7 or 8-hydroxynaphthyl, 2, 4-dihydroxynaphthyl, the protected-hydroxy derivatives thereof and the like; a nitronaphthyl group such as 3- or 4-nitronaphthyl; a cyanonaphthyl group, for example, 1, 2, 3, 4, 5, 6, 7 or 8-cyanonaphthyl; a mono- or di(alkyl)naphthyl group such as 2, 3, 4, 5, 6, 7 or 8-methylnaphthyl, 1, 2, 4-dimethylnaphthyl, 1, 2, 3, 4, 5, 6, 7 or 8-(isopropyl)naphthyl, 1, 2, 3, 4, 5, 6, 7 or 8-ethylnaphthyl, 1, 2, 3, 4, 5, 6, 7 or 8-(n-propyl)naphthyl and the like; a mono or di(alkoxy)naphthyl group, for example, 2, 6-dimethoxynaphthyl, 1, 2, 3, 4, 5, 6, 7 or 8-methoxynaphthyl, 1, 2, 3, 4, 5, 6, 7 or 8-ethoxynaphthyl, 1, 2, 3, 4, 5, 6, 7 or 8-(isopropoxy)naphthyl, 1, 2, 3, 4, 5, 6, 7 or 8-(t-butoxy)naphthyl, 3-ethoxy-4-methoxynaphthyl and the like; 1, 2, 3, 4, 5, 6, 7 or 8-trifluoromethylnaphthyl; a mono- or di-carboxynaphthyl or (protected carboxy)naphthyl group such as 1, 2, 3, 4, 5, 6, 7 or 8-carboxynaphthyl or 2, 4-di(-protected carboxy)naphthyl; a mono-or di(hydroxymethyl)naphthyl or (protected hydroxymethyl)naphthyl such as 1, 2, 3, 4, 5, 6, 7 or 8-(protected hydroxymethyl)naphthyl or 3, 4-di(hydroxymethyl)naphthyl; a mono- or di(amino)naphthyl or (protected amino)naphthyl such as 1, 2, 3, 4, 5, 6, 7 or 8-(amino)naphthyl or 2, 4-(protected amino)-naphthyl, a mono- or di(aminomethyl)naphthyl or (protected aminomethyl)naphthyl such as 2, 3, or 4-(aminomethyl)naphthyl or 2, 4-(protected aminomethyl)-naphthyl; or a mono- or di-(N-methylsulfonylamino) naphthyl such as 1, 2, 3, 4, 5, 6, 7 or 8-(N-methylsulfonylamino)naphthyl. Also, the term "substituted naphthyl" represents disubstituted naphthyl groups wherein the substituents are different, for example, 3-methyl-4-hydroxynaphth-1-yl, 3-chloro-4-hydroxynaphth-2-yl, 2-methoxy-4-bromonaphth-1-yl, 4-ethyl-2-hydroxynaphth-1-yl, 3-hydroxy-4-nitronaphth-2-yl, 2-hydroxy-4-chloronaphth-1-yl, 2-methoxy-7-bromonaphth-1-yl, 4-ethyl-5-hydroxynaphth-2-yl, 3-hydroxy-8-nitronaphth-2-yl, 2-hydroxy-5-chloronaphth-1-yl and the like.

The term "C₁ to C₈ alkyl" denotes such radicals as methyl, ethyl, n-propyl, isopropyl, n-butyl, iso-butyl, sec-butyl, tert-butyl, amyl, tert-amyl, hexyl , n-heptyl, 2-heptyl, 3-heptyl, 4-heptyl, 2-methyl-lhexyl, 2-methyl-2hexyl, 2-methyl-3-hexyl, n-octyl and the like.

The term "C₁ to C₈ substituted alkyl" denotes that the above C₁ to C₈ alkyl groups are substituted by one or more, and preferably one or two, halogen, hydroxy, protected hydroxy, oxo, protected oxo, C₃ to C₇ cycloalkyl, naphthyl, amino, protected amino, (monosubstituted)amino, protected (monosubstituted)amino, (disubstituted)amino, guanidino, protected guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted alkyl groups may be substituted once or more, and preferably once or twice, with the same or with different substituents.

Examples of the above substituted alkyl groups include the 2-oxo-prop-1-yl, 3-oxo-but-1-yl, cyanomethyl, nitromethyl, chloromethyl, hydroxymethyl, tetrahydropyranyloxymethyl, trityloxymethyl, propionyloxymethyl, amino, methylamino, aminomethyl, dimethylamino, carboxymethyl, allyloxycarbonylmethyl, allyloxycarbonylaminomethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichloro(n-butyl), 2-aminopropyl, 1-chloroethyl, 2-chloroethyl, 1- bromoethyl, 2-chloroethyl, 1-fluoroethyl, 2-fluoroethyl, 1- iodoethyl, 2-iodoethyl, 1-chloropropyl, 2-chloropropyl, 3- chloropropyl, 1-bromopropyl, 2-bromopropyl, 3-bromopropyl, 1-fluoropropyl, 2-fluoropropyl, 3-fluoropropyl, 1- iodopropyl, 2-iodopropyl, 3-iodopropyl, 2-aminoethyl, 1- aminoethyl, N-benzoyl-2-aminoethyl, N-acetyl-2-aminoethyl, N-benzoyl-1-aminoethyl, N-acetyl-1-aminoethyl and the like.

The term "C₇ to C₁₂ phenylalkyl" denotes a C₁ to C₆ alkyl group substituted at any position by a phenyl, substituted phenyl, heteroaryl or substituted heteroaryl. Examples of such a group include benzyl, 2-phenylethyl, 3-phenyl(n-propyl), 4-phenylhexyl, 3-phenyl(n-amyl), 3-phenyl(sec-butyl) and the like. Preferred C₇ to C₁₂ phenylalkyl groups are the benzyl and the phenylethyl groups.

The term "C₇ to C₁₂ substituted phenylalkyl" denotes a C₇ to C₁₂ phenylalkyl group substituted on the C₁ to C₆ alkyl portion with one or more, and preferably one or two, groups chosen from halogen, hydroxy, protected hydroxy, oxo, protected oxo, amino, protected amino, (monosubstituted)amino, protected (monosubstituted)amino, (disubstituted)amino, guanidino, protected guanidino, heterocyclic ring, substituted heterocyclic ring, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N, N-(C₁ to C₆ dialkyl)carboxamide, cyano, N-(C₁ to C₆ alkylsulfonyl)amino, thiol, C₁ to C₄ alkylthio, C₁ to C₄ alkylsulfonyl groups; and/or the phenyl group may be substituted with one or more, and preferably one or two, substituents chosen from halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl) carboxamide, protected N-(C₁ to C₆ alkyl) carboxamide, N, N-di(C₁ to C₆ alkyl)carboxamide, trifluoromethyl, N-((C₁ to C₆ alkyl)sulfonyl)amino, N-(phenylsulfonyl)amino, cyclic C₂ to C₇ alkylene or a phenyl group, substituted or unsubstituted, for a resulting biphenyl group. The substituted alkyl or phenyl groups may be substituted with one or more, and preferably one or two, substituents which can be the same or different.

Examples of the term "C₇ to C₁₂ substituted phenylalkyl" include groups such as 2-phenyl-1-chloroethyl, 2-(4-methoxyphenyl)ethyl, 4-(2,6-dihydroxy phenyl)n-hexyl, 2-(5-cyano-3-methoxyphenyl)n-pentyl, 3-(2,6-dimethylphenyl)n-propyl, 4-chloro-3-aminobenzyl, 6-(4-methoxyphenyl)-3-carboxy(n-hexyl), 5-(4-aminomethylphenyl)- 3-(aminomethyl)n-pentyl, 5-phenyl-3-oxo-n-pent-1-yl and the like.

The term "heterocycle" or "heterocyclic ring" denotes optionally substituted three-membered to eight-membered rings that have 1 to 4 heteroatoms, such as oxygen, sulfur and/or nitrogen, in particular nitrogen, either alone or in conjunction with sulfur or oxygen ring atoms. These three-membered to eight-membered rings may be saturated, fully unsaturated or partially unsaturated, with fully saturated rings being preferred. Preferred heterocyclic rings include morpholino, piperidinyl, piperazinyl, 2-amino-imidazoyl, tetrahydrofurano, pyrrolo, tetrahydrothiophen-yl, hexylmethyleneimino and heptylmethyleneimino.

The term "substituted heterocycle" or "substituted heterocyclic ring" means the above-described heterocyclic ring is substituted with, for example, one or more, and preferably one or two, substituents which are the same or different which substituents can be halogen, hydroxy, protected hydroxy,oxo, protected oxo, cyano, nitro, C₁ to C₁₂ alkyl, C₁ to C₁₂ alkoxy, C₁ to C₁₂ substituted alkoxy, C₁ to C₁₂ acyl, C₁ to C₁₂ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino carboxamide, protected carboxamide, N-(C₁ to C₁₂ alkyl)carboxamide, protected N-(C₁ to C₁₂ alkyl)carboxamide, N, N-di(C₁ to C₁₂ alkyl)carboxamide, trifluoromethyl, N-((C₁ to C₁₂ alkyl)sulfonyl)amino, N-(phenylsulfonyl)amino, heterocycle or substituted heterocycle groups.

The term "C₁ to C₈ alkoxy" as used herein denotes groups such as methoxy, ethoxy, n-propoxy, isopropoxy, n-butoxy, t-butoxy and like groups. A preferred alkoxy is methoxy. The term "C₁ to C₈ substituted alkoxy" means the alkyl portion of the alkoxy can be substituted in the same manner as in relation to C₁ to C₈ substituted alkyl. [Please check carefully...]

The term "C₁ to C₈ aminoacyl" encompasses groups such as formyl, acetyl, propionyl, butyryl, pentanoyl, pivaloyl, hexanoyl, heptanoyl, octanoyl, benzoyl and the like attached to a nitrogen moiety.

The term "C₁ to C₈ substituted aminoacyl" denotes the acyl group, attached to a nitrogen moiety, substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₁₂ alkoxy, C₁ to C₁₂ acyl, C₁ to C₁₂ acyloxy, nitro, C₁ to C₁₂ alkyl ester, carboxy, protected carboxy, carbamoyl, carboxamide, protected carboxamide, N-(C₁ to C₁₂ alkyl)carboxamide, protected N-(C₁ to C₁₂ alkyl)carboxamide, N,N-di(C₁ to C₁₂ alkyl)carboxamide, cyano, methylsulfonylamino, thiol, C₁ to C₁₀ alkylthio or C₁ to C₁₀ alkylsulfonyl groups. The substituted acyl groups may be substituted once or more, and preferably once or twice, with the same or with different substituents.

Examples of C₁ to C₈ substituted acyl groups include 4-phenylbutyroyl, 3-phenylbutyroyl, 3-phenylpropanoyl, 2- cyclohexanylacetyl, cyclohexanecarbonyl, 2-furanoyl and 3-dimethylaminobenzoyl.

This invention provides a pharmaceutical composition comprising an effective amount of a compound according to the invention. Such compounds can be administered by various routes, for example oral, subcutaneous, intramuscular, intravenous or intracerebral. The preferred route of administration would be oral at daily doses of the compound for adult human treatment of about 0.01 -5000 mg, preferably 1-1500 mg per day. The appropriate dose may be administered in a single dose or as divided doses presented at appropriate intervals for example as two, three four or more subdoses per day.

For preparing pharmaceutical compositions containing compounds of the invention, inert, pharmaceutically acceptable carriers are used. The pharmaceutical carrier can be either solid or liquid. Solid form preparations include, for example, powders, tablets, dispersible granules, capsules, cachets, and suppositories.

A solid carrier can be one or more substances which can also act as diluents, flavoring agents, solubilizers, lubricants, suspending agents, binders, or tablet disintegrating agents; it can also be an encapsulating material.

In powders, the carrier is generally a finely divided solid which is in a mixture with the finely divided active component. In tablets, the active compound is mixed with the carrier having the necessary binding properties in suitable proportions and compacted in the shape and size desired.

For preparing pharmaceutical composition in the form of suppositories, a low-melting wax such as a mixture of fatty acid glycerides and cocoa butter is first melted and the active ingredient is dispersed therein by, for example, stirring. The molten homogeneous mixture is then poured into convenient-sized molds and allowed to cool and solidify.

Powders and tablets preferably contain between about 5% to about 70% by weight of the active ingredient. Suitable carriers include, for example, magnesium carbonate, magnesium stearate, talc, lactose, sugar, pectin, dextrin, starch, tragacanth, methyl cellulose, sodium carboxymethyl cellulose, a low-melting wax, cocoa butter and the like.

The pharmaceutical compositions can include the formulation of the active compound with encapsulating material as a carrier providing a capsule in which the active component (with or without other carriers) is surrounded by a carrier, which is thus in association with it. In a similar manner, cachets are also included. Tablets, powders, cachets, and capsules can be used as solid dosage forms suitable for oral administration.

Liquid pharmaceutical compositions include, for example, solutions suitable for oral or parenteral administration, or suspensions, and emulsions suitable for oral administration. Sterile water solutions of the active component or sterile solutions of the active component in solvents comprising water, ethanol, or propylene glycol are examples of liquid compositions suitable for parenteral administration.

Sterile solutions can be prepared by dissolving the active component in the desired solvent system, and then passing the resulting solution through a membrane filter to sterilise it or, alternatively, by dissolving the sterile compound in a previously sterilised solvent under sterile conditions.

In one embodiment of the present invention a compound is provided according to formula (2) below, or pharmaceutically acceptable salts or solvates thereof, wherein R₂ is H, C₁ to C₈ alkyl, C₁ to C₈ substituted alkyl, C₇ to C₁₂ alkylphenyl or C₇ to C₁₂ substituted phenylalkyl, R₄ is H, C₁ to C₈ alkyl, halogen, C₁ to C₈ alkoxy, carboxy, ester, amide or C₁ to C₈ aminoacyl, and R₅ is H, C₁ to C₈ alkyl, halogen, C₁ to C₈ alkoxy, carboxy, ester, amide or C₁ to Cg aminoacyl.

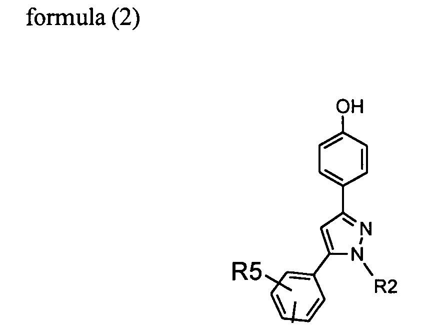

In a preferred embodiment of the invention a compound which may act as an antagonist of NR3B1 according to formula (3) is provided, or pharmaceutical acceptable salts or solvates thereof, wherein R₂ is H, C₁ to C₇ acyl or C₁ to C₇ substituted acyl, phenyl, substituted phenyl, C₅ to C₆ heteroaryl, C₅ to C₆ substituted heteroaryl, napthyl or substituted napthyl, and R₆ is H, C₁ to C₈ alkyl, C₁ to Cg substituted alkyl, C₇ to C₁₂ alkylphenyl or C₇ to C₁₂ substituted phenylalkyl, carboxy, ester, amide or C₁ to C₈ aminoacyl.

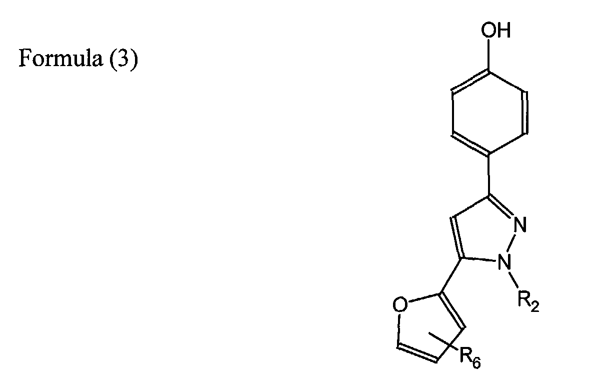

An other preferred compound which may act as antagonist of NR3B1 is shown in formula (4) below. The inventors have been able to demonstrate that the compound according to formula (4), wherein R₂ is H, C₁ to C₇ acyl or C₁ to C₇ substituted acyl, phenyl, substituted phenyl, C₅ to C₆ heteroaryl, C₅ to C₆ substituted heteroaryl, napthyl or substituted napthyl, and R₆ is H, C₁ to C₈ alkyl, C₁ to C₈ substituted alkyl, C₇ to C₁₂ alkylphenyl or C₇ to C₁₂ substituted phenylalkyl, carboxy, ester, amide or C₁ to C₈ aminoacyl has a low effective concentration at NR3B1 with an EC₅₀ of xxx µM wherein the EC₅₀ reflects the half-maximal effective concentration, and which is higher than the EC₅₀ of 1 µM for the published ERR antagonist DES (Tremblay GB et al., Genes & Development 15, 833-838, 2001).

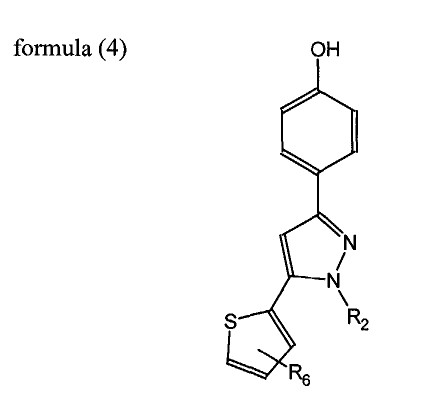

The inventors have also found the compounds according to formula (5 and 6) (shown below) to be active as antagonist of the NR3B1 human nuclear receptor (see figures for details).

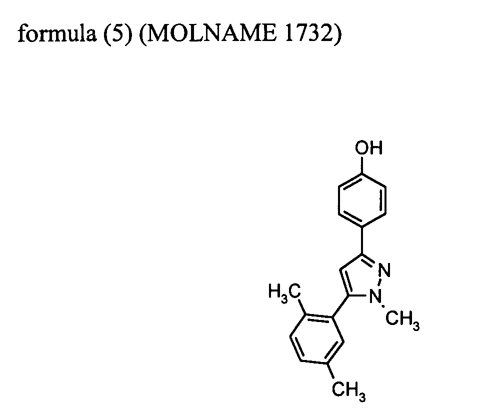

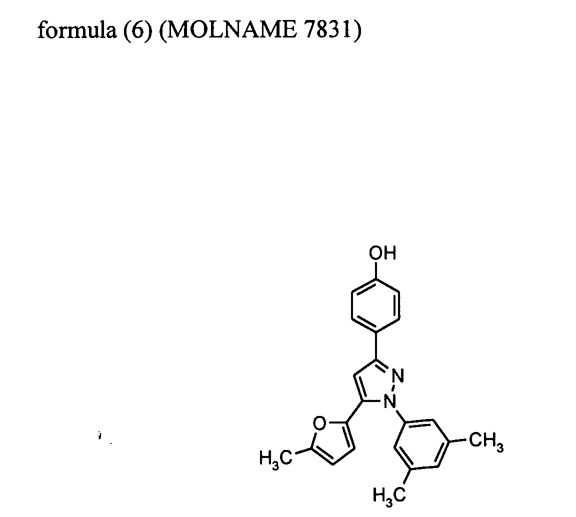

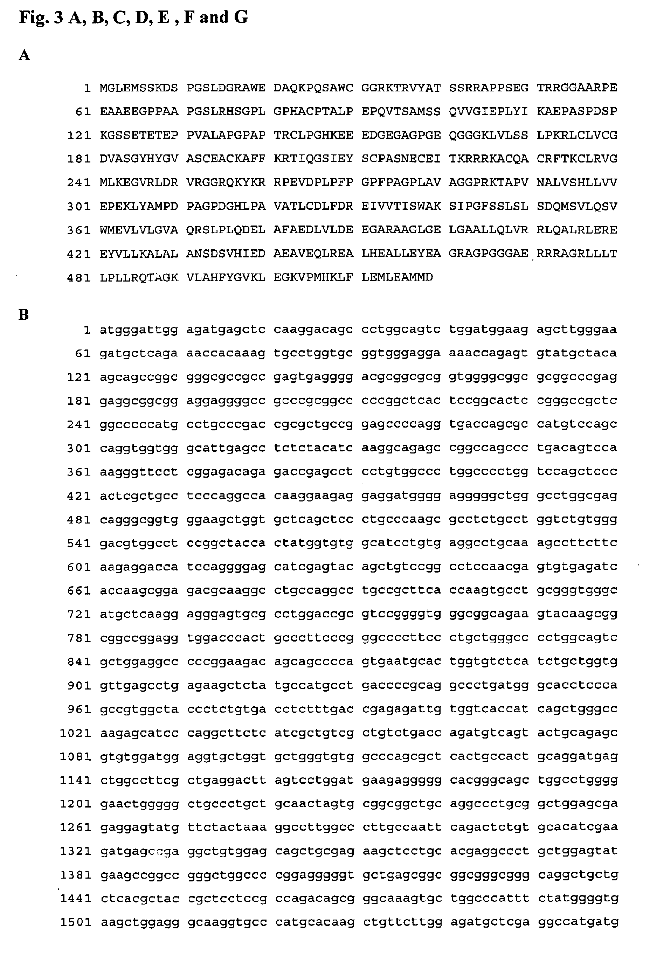

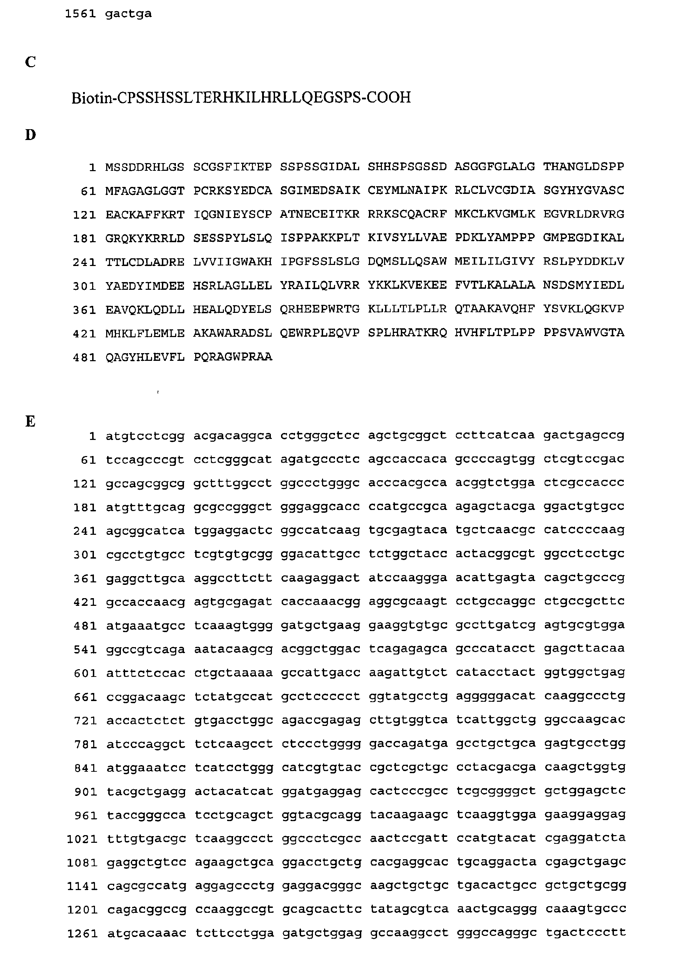

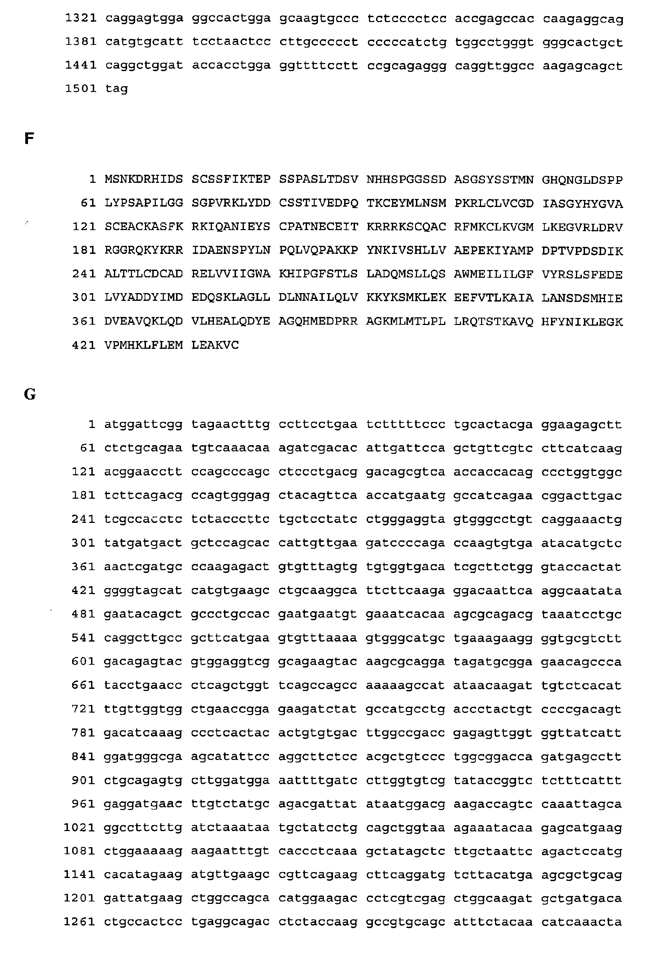

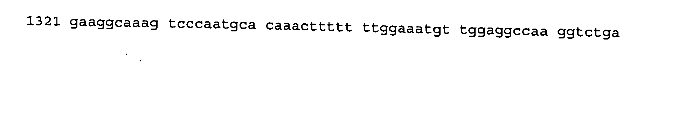

The present invention relates to a compound as described above wherein said compounds is capable of binding the NR3B1 receptor protein or a portion thereof according to SEQ ID NO. 1 (cf. Fig. 3 A to D) or a mammalian homologue thereof. The claimed compound can bind to the NR3B1 receptor protein or a portion thereof in a mixture comprising 10-200 ng of NR3B1 receptor protein or a portion thereof, preferably the ligand binding domain, 20 mM Tris /HCl at pH 7.9; 60 mM KCl; 5 mM MgCl₂; 160ng/µl BSA in a total volume of preferably about 25 µl.

"A mammalian receptor protein homologue" of the protein according to SEQ ID NO. 1 as used herein is a protein that performs substantially the same task as NR3B1 does in humans and shares at least 40% sequence identity at the amino acid level, preferably 50 % sequence identity at the amino acid level more preferably 65 % sequence identity at the amino acid level, even more preferably 75 % sequence identity at the amino acid level and most preferably over 85 % sequence identity at the amino acid level.

The invention in particular relates to a method for prevention or treatment of a NR3B1 receptor protein or NR3B1 receptor protein homologue mediated disease or condition in a mammal comprising administration of a therapeutically effective amount of a compound according to the invention wherein the prevention or treatment is directly or indirectly accomplished through the binding of a compound according to the invention to the NR3B1 receptor protein or to the NR3B1 receptor protein homologue.

The term "mediated" herein means that the physiological pathway in which the NR3B1 receptor protein acts is either directly or indirectly involved in the disease or condition to be treated or prevented. In the case where it is indirectly involved it could be that, e.g. modulating the activity of NR3B1 by a compound according to the invention influences a parameter which has a beneficial effect on a disease or a condition. One such example is that modulation of NR3B activity leads to decreased levels of aromatase levels which in turn have a beneficial effect on the prevention and treatment of mammary cancer. Herein a condition is a physiological or phenotypic state which is desirably altered. One such example would be obesity which is not necessarily medically harmful but nonetheless a non desirable phenotypic condition. In a preferred embodiment of the invention the method for prevention or treatment of a NR3B1 receptor protein mediated disease or condition is applied to a human. This may be male or female.

Pharmaceutical compositions generally are administered in an amount effective for treatment or prophylaxis of a specific condition or conditions. Initial dosing in human is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, doses is from about 100 µg/kg to about 5 mg/kg body weight, daily.

For administration particularly to mammals, and particularly humans, it is expected that the daily dosage level of active agent will be 0,1 mg/kg to 10 mg/kg and typically around 1 mg/kg.

By "therapeutically effective amount" is meant a symptom- alleviating or symptom -reducing amount, a cholesterol-reducing amount, a fatty acid absorption blocking amount, a protein and/or carbohydrate digestion-blocking amount, a MCAD modulating amount or a thyroid receptor modulating amount or a osteopontin modulating amount and/or a aromatase modulating amount of a compound according to the invention.

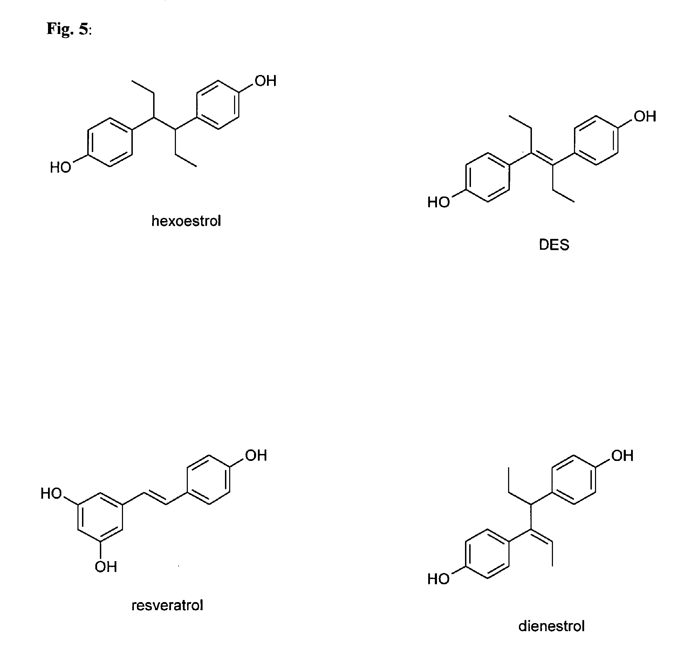

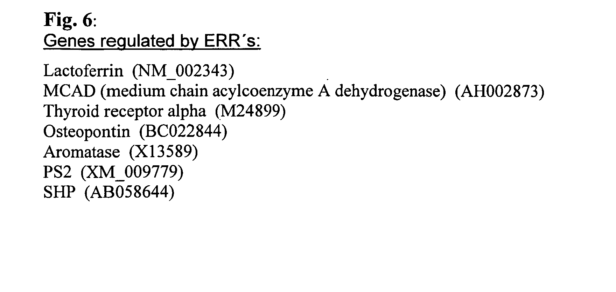

The Estrogen receptor related receptor alpha is a nuclear receptor which modulates genes upon binding to the promoter region of target genes in a homodimeric or monomeric fashion or as heterodimers with the ERa. The relevant physiological ligands of NR3B1 are unknown. The present compounds according to the invention have been demonstrated to have a high binding efficacy [binding coefficients measured as IC50 in the range 1 to 10µM] as well as antagonistic and / or agonistic properties. Consequently they may be applied to regulate genes that participate in estrogen regulated gene networks as well as other downstream regulated genes. Examples of such genes are Lactoferrin, MCAD, Aromatase, PS2 and SHP and the function of such genes is associated but not limited to cancer, osteogenesis, obesity, energy metabolism, lipid absorption, cholesterol biosynthesis, cholesterol transport or binding, bile acid transport or binding, proteolysis, amino acid metabolism, glucose biosynthesis, protein translation, electron transport, and hepatic fatty acid metabolism. The ERR's often function in vivo as homodimers or as monomers. Published ERR antagonists such as the DES (See figure 5) are known to influence the regulation of various genes. Genes found to be regulated by DES can be found in figure 6. Thus, the invention also concerns a method of modulating a gene whose expression is regulated by the NR3B1 receptor in a mammal comprising administration of a therapeutically effective amount of a compound according to the invention to said mammal.

It is known that the orphan receptor ERRa can bind the response element of the aromatase gene (Yang C 2002 Oncogene 21, 2854-2863). Other genes controlled via ERRa comprise the medium chain acetyl dehydrogenase MCAD , Thyroid receptor alpha, osteopontin, PS2 and lactoferrin.

The compounds according to the invention may be used as medicaments, in particular for the manufacture of a medicament for the prevention or treatment of a NR3B1 receptor protein or NR3B1 receptor protein homologue mediated disease or condition in a mammal wherein the prevention or treatment is directly or indirectly accomplished through the binding of the compound according to the invention to the NR3B1 receptor protein or NR3B1 receptor protein homologue. These pharmaceutical compositions contain 0,1 % to 99,5 % of the compound according to the invention, more particularly 0,5 % to 90 % of the compound according to the invention in combination with a pharmaceutically acceptable carrier.

The invention relates also to the use of a compound according to the invention for the manufacture of a medicament for the prevention or treatment of a NR3B1 receptor protein mediated disease or condition wherein the mammal described above is a human. The medicament may be used for regulating the growth of cancer cells in a mammal preferentially a human by modulating the NR3B1 receptor, for regulating cancer, osteoporosis, lipid disorders or a cardiovascular disorder in humans or influencing fertility and reproductive health.

The further concerns the use of a compound according to the invention for the manufacture of a medicament capable for blocking in a mammal, preferentially a human the fatty acid absorption in the intestine. Further the claimed compound may be used for the manufacture of a medicament for treating obesity in humans and for modulating a gene whose expression is regulated by the NR3B1 receptor (see details above and figures). The invention further concerns the use of a compound according to the invention for the manufacture of antitumor medicaments. The antitumor effects of such medicaments could be excerted by selective inhibition of cell proliferation and induction of apotptosis of tumour cells.

The invention shall now be further described in the following examples, without being limited thereto. In the accompanying figure and the sequence protocol,
SEQ ID NO. 1: shows the peptide sequence of ERRa,
SEQ ID NO. 2: shows the nucleotide sequence of the region that encodes for ERRa,
SEQ ID NO. 3: depicts the sequence of the biotinylated SRC peptide,
SEQ ID NO. 4: shows primer ERRE_1, used in Examples 3 and 4,
SEQ ID NO. 5: shows primer ERRE_2, used in Examples 3 and 4,
SEQ ID NO 6 is the protein sequence of the ERRb protein,
SEQ ID NO 7 is the mRNA sequence coding for the ERRb protein,
SEQ ID NO 7 is the protein sequence of the ERRg protein, and
SEQ ID NO 8 is the mRNA sequence of the ERRg protein.

Fig. 1 shows the synthesis of the compounds according to the invention as also described in Example 2.

Fig. 2 shows the measurement parameters employed by the Wallace VICTOR2V™ Multilabel Counter which was used for measuring the EC₅₀ values

Figure 3 A shows SEQ ID NO. 1 which is the protein sequence of the ERRa protein a portion of which was used for cloning as described in the examples. Figure 3 B shows SEQ ID NO. 2 which is the mRNA sequence of the ERRa protein. Figure 3 C shows SEQ ID NO. 3 which is the sequence of the biotinylated SRC1 peptide. Figure 3D shows SEQ ID NO 4 which is the protein sequence of the ERRb protein a portion of which was used according to the example described for ERRa. Figure 3E shows SEQ ID NO 5 which is the mRNA sequence coding for the ERRb protein. Figure 3F show SEQ ID NO 6 which is the protein sequence of the ERRg protein, a portion of which was used for cloning according to the example described for ERRa. Figure 3G shows SEQ ID NO 7 which is the mRNA sequence of the ERRg protein.

Fig. 4 shows the internal molecular name used by the applicant (MOLNAME) as well as the corresponding structures of preferred compounds according to the invention. The figure further shows their respective EC₅₀ values (EC50 AVG) as established according to the example 1 in multiple experiments (see above), as well as their respective estimated average efficacy (% activity relative to DES control antagonist).

Figure 5 shows various known ERRa ligands. It is apparent from their structures that the inventors have identified novel compounds which are structurally not related to these known ligands.

Figure 6 shows various genes that have been found to be regulated through binding of an ERRa protein.

Figure 7 shows a dose-dependent transrepression (EC50 ∼ 1-5 µM) of the ERRa reporter gene by ERRa by 01723 (A) or 07831 (B).

Figure 8 shows the stimulation of the ERRa driven reporter gene activity by RIP 140 and repression of this RIP140 dependent activity by 10µM 01723, 07831 or DES.

Figure 9 shows the ERRa ligand DES and the compound 01723, inhibit growth of the ER negative breast cancer cell line MDA-MB-231 (Fig. 9A). The compound 01723 does not induce proliferation of the ER positive breast cancer cell line T47D, while both E2 (estradiol) and DES stimulate the growth of T47D cells at indicated concentrations (Fig 9B).

### EXAMPLES

### EXAMPLE 1:

In vitro screening for compounds which influence ERRa binding to coactivators.

For screening purposes a fragment of the open reading frame of human ERR alpha (NR3B1 - (Acc. No: NM_004451 )) encoding aminoacids 187-472 was amplified by standard RT PCR procedures (see figures; SEQ ID NO. 1 and 2). Starting material was total RNA derived from human uterus. The resulting cDNA obtained after reverse transcription was subsequently cloned using the Gateway™ recombination technology (Invitrogen, USA) into the expression plasmid pDest15 (Invitrogen, USA). This construct was used to express a recombinant GST-ERRa fusion protein in E.coli (BL21 strain). For E. coli expression of both constructs, plasmid DNA was transformed into chemically competent E. coli BL21 (Invitrogen, USA) and cells were grown to an OD600 of 0.4-0.7 before expression was induced by addition of 0,5 mM IPTG according instructions of the manufacturer (Invitrogen). After induction for 8 hours at 30°C cells were harvested by centrifugation for 10 minutes at 5000 x g. Fusion proteins were affinity purified using Glutathion sepharose (Pharmacia) or Ni-NTA Agarose (QIAGEN) according to the instructions of the respective manufacturer. Recombinant proteins were dialyzed against 20 mM Tris/HCL pH 7.9; 60 mM KCl; 5 mM MgCl₂; 1 mM DTT, 0,2 mM PMSF; 10% glycerol.

For screening of compound libraries as provided for by the methods shown below in the examples for substances which influence the ERRa/SRC interaction, the Perkin Elmer LANCE technology was applied. This method relies on the binding dependent energy transfer from a donor to an acceptor fluorophore attached to the binding partners of interest. For ease of handling and reduction of background from compound fluorescence LANCE technology makes use of generic fluorophore labels and time resoved detection (for detailed description see Hemmilä I, Blomberg K and Hurskainen P, Time-resolved resonance energy transfer (TR-FRET) principle in LANCE, Abstract of Papers Presented at the 3 rd Annual Conference of the Society for Biomolecular Screening, Sep., California (1997)).

For screening, 80 ng of biotinylated SRC1 peptide and 10-200 ng of GST-ERRa fragment were combined with 0.5-2 nM LANCE Eu-(W1024) labelled anti-GST antibody (Perkin Elmer) and 0,5-2µg of Highly fluorescent APC-labelled streptavidin (Perkin Elmer) in the presence of 50µM of individual compounds to be screened in a total volume of 25 µl of 10 mM Hepes pH 7.9; 550 mM NaCl; 2 mM MgCl₂; 40ng/µl BSA. DMSO content of the samples was kept below 1 %. Samples were incubated for a minimum of 60 minutes in the dark at room temperature in FIA-Plates black 384well med. binding (Greiner).

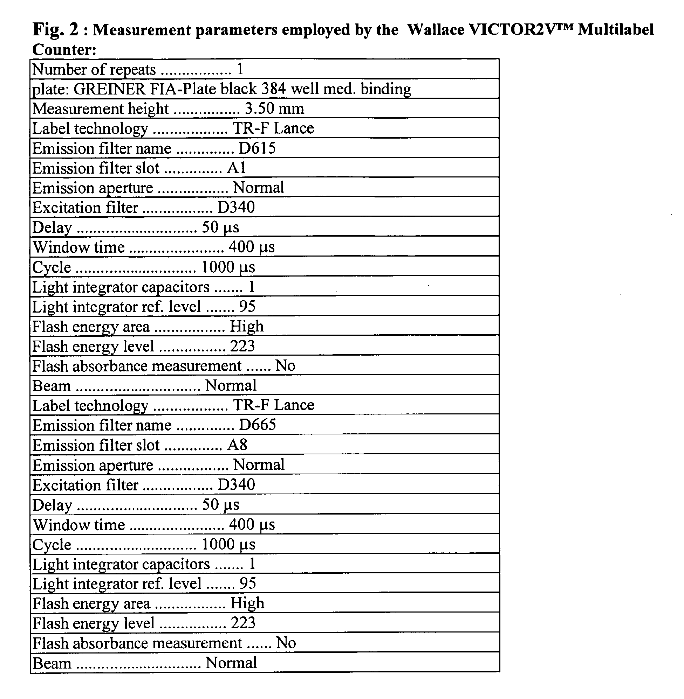

The LANCE signal was detected by a Perkin Elmer VICTOR2V™ Multilabel Counter applying the detection parameters listed in Fig. 2. The results were visualized by plotting the ratio between the emitted light at 665 nm and at 615 nm. For every batch of recombinant proteins amount of proteins and labeling reagents giving the most sensitive detection of hits was determined individually by analysis of dose response curves for DES.

### EXAMPLE 2:

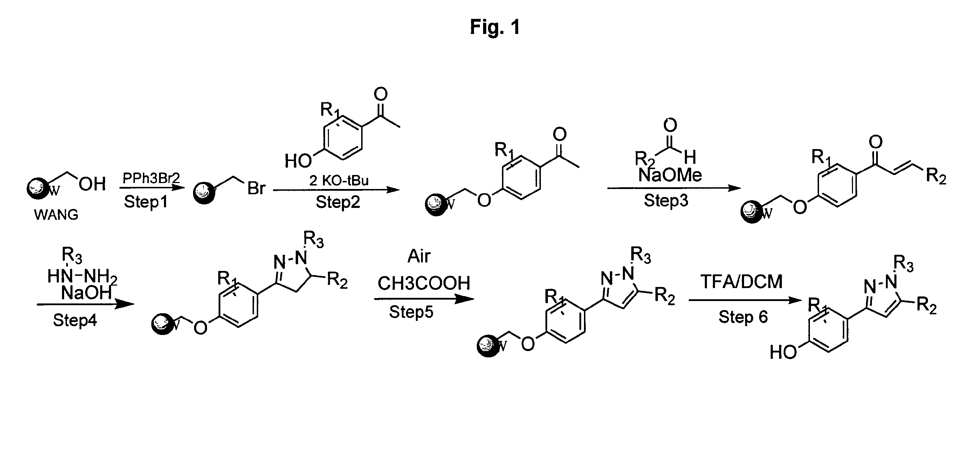

Experimental procedure for the preparation of the compounds according to the invention (see also Fig 1).

### Step 1. General procedure for preparation of Br-Wang resin

1.6 g of Wang resin (1.28 mmol/g, 2.0 mmol/bag) was placed in a porous polypropylene packets (Tea-bag, 60 mm x 50 mm, 65 µ), sealed and transferred to a 125 ml PP bottle. A freshly prepared solution of PPh₃Br₂ (6.1 mmol, 3.0 equivalents, 0.15 M) in DCM (40 mL) was added to each packet. After shaking for 4-6 hours at room temperature, the packet was washed with DCM (5x80 ml) and diethyl ether (4x80 ml). The packet was dried overnight under vacuum to afford off-white resin.

### Step 2. Reaction of Acetophenones with Br-Wang resin.

Each packet containing freshly prepared Br-Wang resin was transferred to an appropriate glass bottle, to which an Acetophenone (20 mmol, 10 equivalents, 0.2 M), anhydrous DMA (100 ml) and KOtBu (20 mmol, 10 equivalents, 0.2 M) were added sequentially. After heating at 50°C for 24 hours, the packet was washed alternatively with DMF (3x80 ml) and MeOH (2x80 ml) followed by DCM (2x80 ml) and MeOH (3x80 ml). The packet was air-dried overnight to afford off-white to pale brown resin, depending on the Acetophenone used in the synthesis.

### Step 3. Reaction of Aldehydes with Wang resin-bound Acetophenones.

Each packet of Acetophenone-Wang resin was transferred to a 250 mL PP bottle, to which a solution of NaOMe (40 mmol, 20 eq, 0.25 M) in 50%THF-MeOH (160 ml) and an Aldehyde (40 mmol, 20 equivalents, 0.25 M ) were added sequentially. After shaking at room temperature for 3 days, the packet was washed several times with MeOH (3x80 ml) and alternatively with DMF (80 ml) and MeOH (80 ml) for 3 cycles, followed by washes of DCM (2x80 ml) and MeOH (3x80 ml). The packet was air-dried overnight to afford a resin-bound chalcone, that varied in color from yellow to dark red depending on the aldehyde used.

### Step 4: Reaction of Hydrazines with Wang resin-bound Chalcone.

The tea bag containing the Chalcone on Wang resin from step was cut open and the resin was equally distributed into 40 wells of a microtiter plate (approx. 50 mg, 0.0575 mmol for each well). The appropriate Hydrazine (0.575 mmol, 10 eq, 0.32 M), NaOH (1.15 mmol, 20 eq, 0.65 M) and MeOEtOH (1.8 mL) were mixed for 2 hrs at room temperature. The supernatant (hydrazine solution) was then added to the corresponding well. The plate was tightly capped, gently shaken and incubated at 75 °C for 24 hours. Each plate was washed alternatively with DMSO (5x 1 mL/well), DMF (6x 1mL/well), and MeOH (8x 1 mL/well). The plate was air-dried overnight and under vacuum for 4 hours.

### Step 5. Air Oxidation of Pyrazoline in acetic acid:

To each well of microtiter plate containing the pyrazoline resins was added 1 ml of acetic acid which was bubbled with air for 20 minutes. The plates were tightly capped , gently shaken , and incubated at 75°C for 48 hours. Each plate was washed alternatively with DMF (6x 1 mL/well) and MeOH (8x 1 mL/well). The plate was air-dried overnight. and under vacuum for 4 hours.

### STEP 6. CLEAVAGE FROM LINKER AND EXTRACTION

To dry microtiter plates containing the pyrazole-resins was added 0.5 mL of 20% TFA/DCM to each well. The plates were capped and placed on a shaker at room temperature for 3 h. The plates were transferred to a GENEVAC to remove the volatile TFA/DCM solution. The plates were extracted with AcOH and the extract frozen and lyophilised to afford the title compounds. All of the final products were analysed by HPLC/MS using ELSD detection to determine purity.

### EXAMPLE 3:

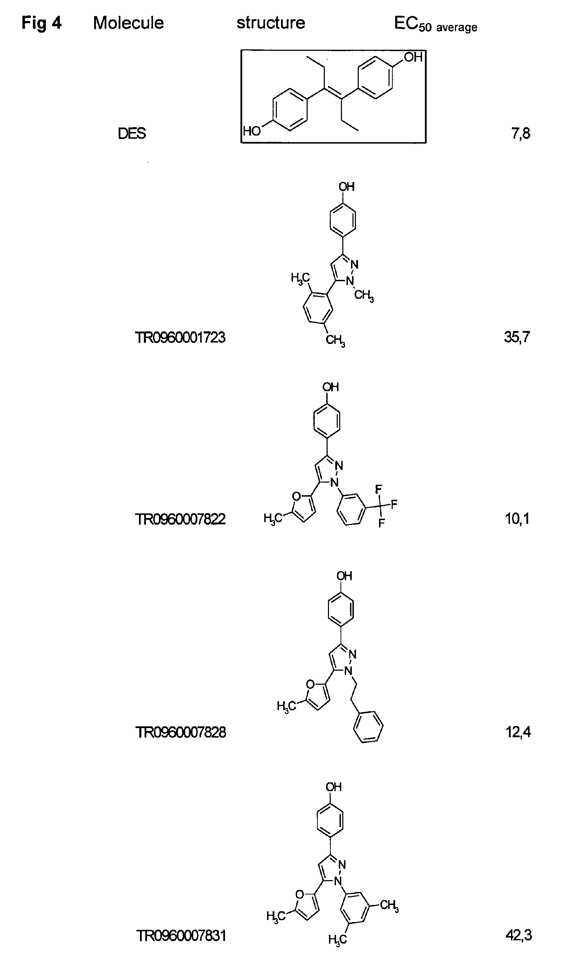

This example illustrates that a compound according to the invention (experiments shown were done with MOLSTRUCTURE 01723 and 07831 (see figure 4 for structural formula) can mediate repression of ERRa mediated transcription in HEK293 cells.

HEK293-were transiently transfected with the pTRexDest30 (Invitrogen) derivatives pTRex-Dest30-hERRa and the pGL2promoter (Promega) derivative pGL2promoter-ERR-RE2. The Renilla-Luciferase pRL-CMV Vector (Promega) was included as a control for transfection efficiency.

The full length human ERRa (accession NM_004451) was cloned into the pTRexDest30 applying the manufacturer protocols for the Gateway™ system (Invitrogen). The pGL2promoter-ERR-RE2 (ERRa reporter gene construct) was generated by annealing oligonucleotides ERRE_1 (5'-TCGAGGCGATTTGTCAAGGTCACACAGTA-3') (SEQ ID No. 4) and ERRE_2 (5'-TCGATACTGTGTGACCTTGACAAATCGCC-3') (SEQ ID No. 5) and cloning them into the Xho I site of the pGL2promoter (Promega). The oligonucleotides contain a consensus ERR response element (underlined). Sequencing confirmed the presence of two copies of the ERR RE.
Luciferase reporter activity was measured in triplicates from extracts of cells after incubating cells in culture medium (phenolred-free DMEM [Gibco-BRL] + 10% charcoal treated FBS [Perbio Science GmbH]) for 16 hours (5% CO₂ 37°C) containing 0,001% DMSO (control) or 0,001 % DMSO with increasing concentrations of 01723 or 07831.

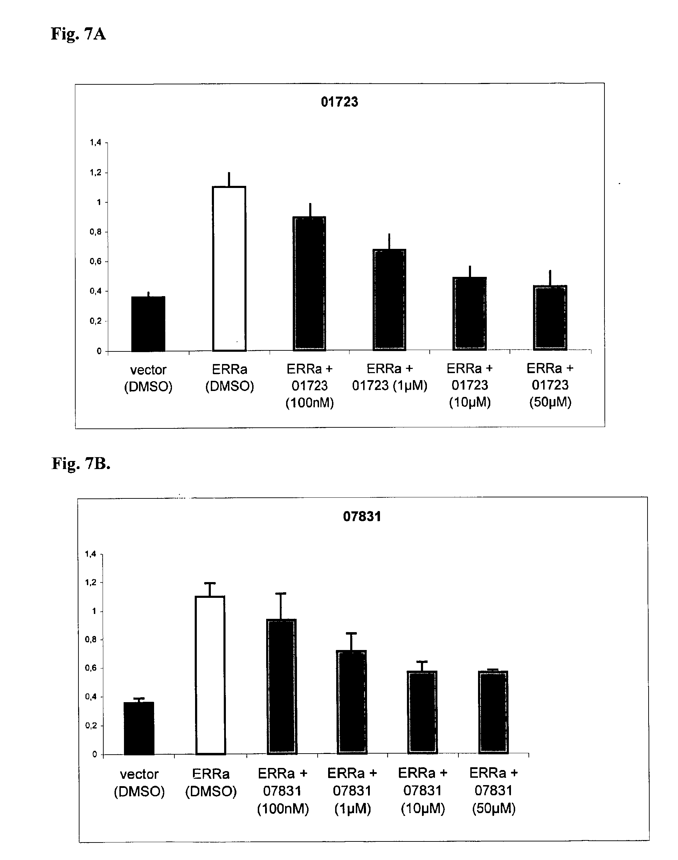

A dose-dependent repression (EC50 ∼ 1-5 µM) of the reporter gene driven by ERRa was observed for the compounds TR0960001723 (Fig. 7A) and TR0960007831 (Fig.7 B). Variations of tripicate measurements are indicated.

### Example 4:

This example illustrates that a compound according to the invention (experiments shown were done with MOLSTRUCTURE TR0960001723 and TR0960007831 and DES as a control (see figure 4 and 5 for structural formula) can mediate repression of ERRa mediated transcription in HEK293 cells.

HEK293-were transiently transfected with the pTRexDest30 (Invitrogen) derivatives pTRex-Dest30-hERRa and pTRexDest30-hRIP140 and the pGL2promoter (Promega) derivative pGL2promoter-ERR-RE2. The Renilla-Luciferase pRL-CMV Vector (Promega) was included as a control for transfection efficiency.

The full length human ERRa (accession NM _004451) and RIP140 (X84373) were cloned into the pTRexDest30 applying the manufacturer protocols for the Gateway™ system (Invitrogen).

The pGL2promoter-ERR-RE2 was generated by annealing oligonucleotides ERRE_1 (5'-TCGAGGCGATTTGTCAAGGTCACACAGTA-3') (SEQ ID No. 4) and ERRE_2 (5'-TCGATACTGTGTGACCTTGACAAATCGCC- 3') (SEQ ID No. 5) and cloning them into the Xho I site of the pGL2promoter (Promega). The oligonucleotides contain a consensus ERR response element (underlined). Sequencing confirmed the presence of two copies of the ERR RE.

Luciferase reporter activity was measured in triplicates from extracts of cells after incubating cells in culture medium (phenolred-free DMEM [Gibco-BRL] + 10% charcoal treated FBS [Perbio Science GmbH]) for 16 hours (5% CO₂, 37°C) containing 0,001% DMSO (control) or 0,001% DMSO with increasing concentrations of 01723 or 07831.

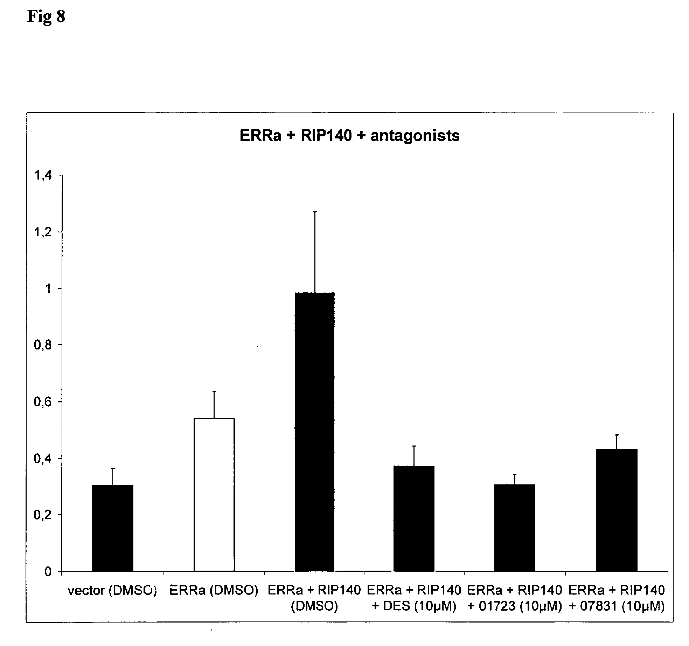

The activity of the ERRa reporter gene construct in HEK293 cells is enhanced when ERRa is cotransfected. This activity is further enhanced, when RIP140 is cotransfected in addition. The observed enhancement of ERRa activity is significantly reduced, when 10 µM of either DES or TR0960001723 or TR0960007831 are added to the medium (see Fig 8)

### EXAMPLE 5:

This example illustrates that a compound according to the patent (MOLSTRUCTURE TR0960001723; see figure 4 for structural formula) can inhibit proliferation of an estrogen receptor negative cell line (MDA-MB-231).

Cell proliferation assays were performed with MDA-MB-231 cells (ER negative) and T47D cells (ER positive) in the presence of 17β-estradiol (E2), DES, TR0960001723 or TR0960007831. Cells were seeded in 96-well plates at a density of 15,000 (MDA-MB-231) or 10,000 (T47D) cells/100 µl/well in phenol red-free DMEM (Gibco-BRL) containing 10% (MDA-MB-231) or 2% (T47D) charcoal dextran treated FBS (Perbio Science GmbH). Treatment media containing either 0,1 % DMSO (control) or 0,1 % DMSO with increasing concentrations of compounds (as indicated) were added on the following day and replaced at 72h-intervals until the end of the experiment. At 6 days (MDA-MB-231) or 8 days (T47D) after initiation of the treatment a colorimetric proliferation assay was performed using CellTiter 96 Aqueous nonradioactive proliferation assay as directed by the manufacturer (Promega). Cell proliferation rates were measured as absorbance of the formazan product at 490 nm. Values are expressed as percentage of control (DMSO) and represent the means of three replicates.

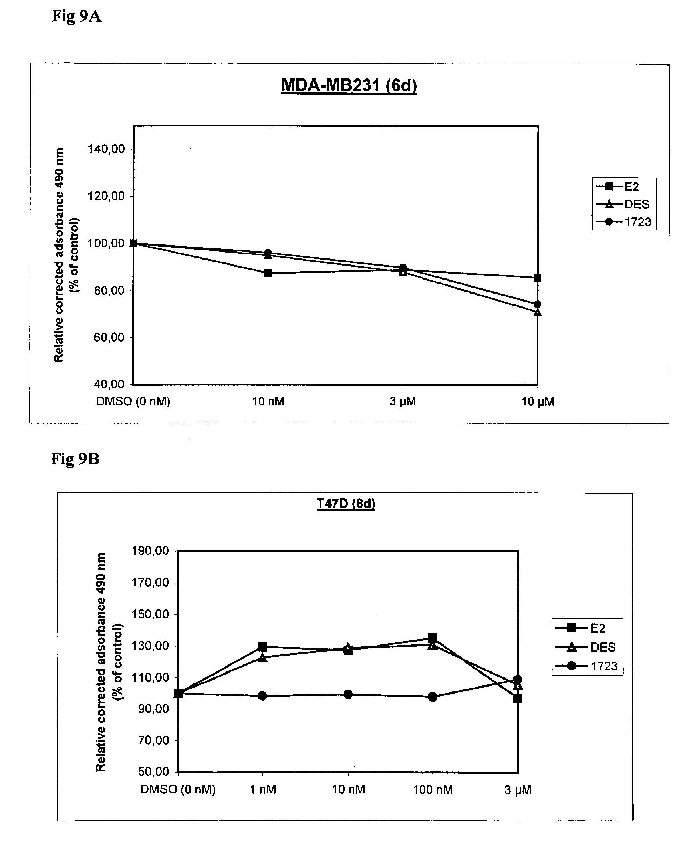

As observed, DES and 17β-estradiol stimulated T47D cell proliferation at 1 nM to 100 nM, but TR0960001723 had no effect (Fig.9B). In contrast, DES and TR0960001723 inhibited proliferation of MDA-MB-231 cells whereas 17β-estradiol did not (Fig 9A).

## Claims

1. A compound according to formula (1), or pharmaceutical acceptable salts or solvates thereof, 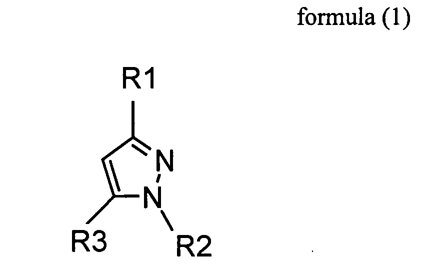 wherein:
R₁ is phenyl, substituted phenyl, C₅ to C₆ heteroaryl, C₅ to C₆ substituted heteroaryl, napthyl or substituted napthyl,
R₂ is H, C₁ to C₈ alkyl, C₁ to C₇ acyl or C₁ to C₇ substituted acyl, C₁ to C₈ substituted alkyl, C₇ to C₁₂ alkylphenyl or C₇ to C₁₂ substituted phenylalkyl, C₃ to C₈ cycloalkyl, C₃ to C₈ substituted cycloalkyl,, C₅ to C₆ heteroaryl, [C₅ to C₆]-heteroaryl-(C₁ to C₆)-alkyl, and
R₃ is H, C₁ to C₈ alkyl, C₁ to C₈ substituted alkyl, C₇ to C₁₂ alkylphenyl or or C₇ to C₁₂ substituted phenylalkyl, halogen, C₁ to C₈ alkoxy, furanyl, substituted furanyl, thiazyl, substituted thiazyl, carboxy, ester, amide or C₁ to Cg aminoacyl.

2. A compound according to claim 1, or pharmaceutical acceptable salts or solvates thereof, wherein:
R₁ is phenyl, or substituted phenyl, C₅ to C₆ heteroaryl, C₅ to C₆ substituted heteroaryl,
R₂ is H, CH₃, substituted alkyl or substituted phenyl, and
R₃ is substitutet phenyl, C₅ to C₆ heteroaryl or C₅ to C₆ substituted heteroaryl.

3. A compound according to claim 2, or pharmaceutical acceptable salts or solvates thereof, wherein:
R₁ is substituted phenyl,
R₂ is CH₃ or substituted alkyl, and
R₃ is substituted phenyl or substituted C₅ heteroaryl.

4. A compound according to claim 1, having the following formula (2) 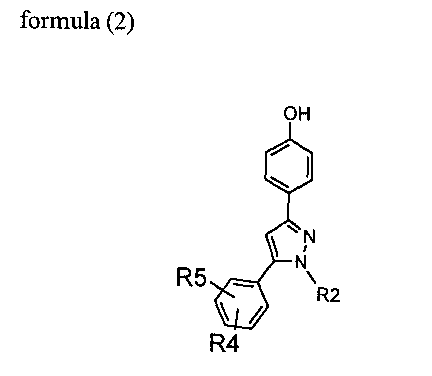 wherein:
R₂ is H, C₁ to C₈ alkyl, C₁ to C₈ substituted alkyl, C₇ to C₁₂ alkylphenyl or C₇ to C₁₂ substituted phenylalkyl,
R₄ is H, C₁ to C₈ alkyl, halogen, C, to C₈ alkoxy, carboxy, ester, amide or C₁ to C₈ aminoacyl, and
R₅ is H, C, to C₈ alkyl, halogen, C₁ to C₈ alkoxy, carboxy, ester, amide or C₁ to C₈ aminoacyl.

5. A compound according to any of claims 1 to 4 having the following formula (3) 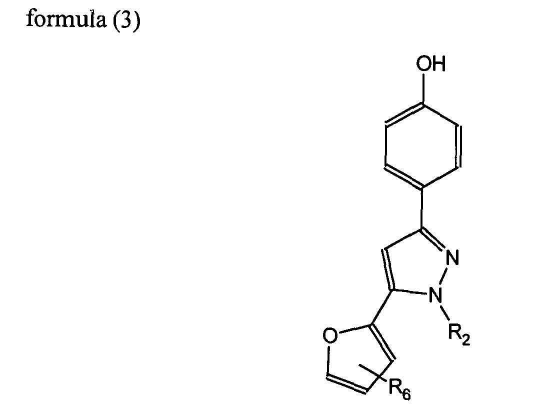 wherein:
R₂ is H, C₁ to C₇ acyl or C₁ to C₇ substituted acyl, phenyl, substituted phenyl, C₅ to C₆ heteroaryl, C₅ to C₆ substituted heteroaryl, napthyl or substituted napthyl,
R₆ is H, C₁ to C₈ alkyl, C₁ to C₈ substituted alkyl, C₇ to C₁₂ alkylphenyl or C₇ to C₁₂ substituted phenylalkyl, carboxy, ester, amide or C₁ to C₈ aminoacyl, C₁ to C₈ alkoxy.

6. A compound according to any of claims 1 to 4 having the following formula (4) 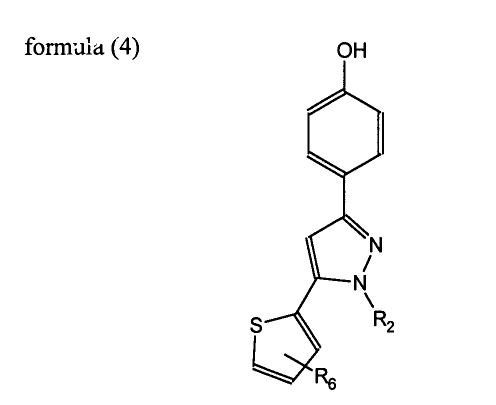 wherein:
R₂ is H, C₁ to C₇ acyl or C₁ to C₇ substituted acyl, phenyl, substituted phenyl, C₅ to C₆ heteroaryl, C₅ to C₆ substituted heteroaryl, napthyl or substituted napthyl,
R₆ is H, C₁ to C₈ alkyl, C₁ to C₈ substituted alkyl, C₇ to C₁₂ alkylphenyl or C₇ to C₁₂ substituted phenylalkyl, carboxy, ester, amide or C₁ to C₈ aminoacyl, C₁ to C₈ alkoxy.

7. A compound according to any of claims 1 to 6 wherein said compound is capable of binding the NR3B1 receptor protein or a portion thereof according to SEQ ID NO. 3 or a mammalian homologue thereof.

8. A compound according to any of claims 1 to 6 wherein said compound is capable of modulating the activity of the NR3B1 receptor protein comprising antagonistic or agonistic effects.

9. A method for prevention or treatment of a NR3B1 receptor protein or NR3B1 receptor protein homologue mediated disease or condition in a mammal comprising administration of a therapeutically effective amount of a compound according to any of claims 1 to 6 wherein the prevention or treatment is directly or indirectly accomplished through the binding of the compound according to any of claims 1 to 8 to the NR3B1 receptor protein or to the NR3B1 receptor protein homologue.

10. A method for prevention or treatment of a NR3B1 receptor protein mediated disease or condition according to claim 9 wherein the mammal is a human.

11. A method for regulating physiologies that are influenced by estrogenic response pathways in a mammal comprising modulating the activity of the NR3B1 receptor with a therapeutically effective amount of a compound according to any of claims 1 to 8.

12. A method of treating in mammal a disease which is directly or indirectly affected by estrogen levels comprising administering to a mammal in need of such treatment a therapeutically effective amount of a compound according to any of claims 1 to 8.

13. A method of treating cancer, osteoporosis, obesity, lipid disorders or a cardiovascular disorder or influencing fertility and reproductive health in a mammal, comprising administering to a mammal in need of such treatment a therapeutically effective amount of a compound according to any of claims 1 to 8.

14. A method of modulating the expression of a gene directly or indirectly controlled by NR3B in tissues of a mammal comprising administering to a mammal in need of such modulation an effective amount of a compound according to any of claims 1 to 8.

15. The method of claim 13 or 14,wherein said mammal is a human.

16. Use of a method according to claim 15 for treating cancer, osteoporosis, lipid disorders or a cardiovascular disorder in humans or influencing fertility and reproductive health.

17. A method according to claim 14 wherein the expression of genes comprising aromatase, MCAD, thyroid receptor alpha, osteopontin, PS2, lactoferrin is modulated.

18. Use of a compound according to any of the claims 1 to 8 as a medicament.

19. Use of a compound according to any of claims 1 to 8 for the manufacture of a medicament for the prevention or treatment of a NR3B1 receptor protein or NR3B1 receptor protein homologue mediated disease or condition in a mammal wherein the prevention or treatment is directly or indirectly accomplished through the binding of the compound according claims 1 to 8 to the NR3B1 receptor protein or NR3B1 receptor protein homologue.

20. Use of a compound according to any of claims 1 to 8 for the manufacture of a medicament for prevention or treatment of a NR3B1 receptor protein mediated disease or condition according to claim 19 wherein the mammal is a human.

21. Use of a compound according to any of claims 1 to 8 for the manufacture of a medicament for regulating estrogenic signaling systems in a mammal by modulating the NR3B1 receptor.

22. Use of a compound according to any of claims 1 to 8 for the manufacture of a medicament for regulating levels of aromatase, bone morphogenic, and/or lipogenic factors or proteins.

23. Use of a compound according to any of claims 1 to 8 for the manufacture of a medicament for treating in a mammal cancer, osteoporosis, obesity, atherosclerosis, lipid disorders or a cardiovascular disorder or influencing fertility and reproductive health.

24. Use of a compound according to any of claims 1 to 8 for the manufacture of a medicament capable for blocking in a mammal the proliferation of estrogen receptor positive and estrogen receptor negative cells, in particular cancer cells.

25. Use of the compound according to claim 24 for the manufacture of a medicament for treating obesity in a mammal, in particular a human.

26. Use of a compound according to any of claims 1 to 8 for the manufacture of a medicament for modulating a gene whose expression is regulated by the NR3B1 receptor.
